# EUROPEAN PATENT SPECIFICATION

(11) **EP 4 014 915 B1**
(45) Date of publication and mention of the grant of the patent: **01.05.2024**
(21) Application number: 20871955.9
(22) Date of filing: 09.06.2020
(51) Int. Cl.: A61B 34/20, A61F 2/46, A61B 17/17, A61B 17/15, A61B 90/00, A61B 34/30

(54) **POSITION CORRECTION METHOD OF OSTEOTOMY GUIDE TOOL, AND ORTHOPEDIC SURGERY SYSTEM**
POSITIONSKORREKTURVERFAHREN FÜR OSTEOTOMIEFÜHRUNGSWERKZEUG UND ORTHOPÄDISCHES CHIRURGISCHES SYSTEM
PROCÉDÉ DE CORRECTION DE POSITION D'OUTIL DE GUIDAGE D'OSTÉOTOMIE, ET SYSTÈME DE CHIRURGIE ORTHOPÉDIQUE

(30) Priority: 30.09.2019 CN 201910940234
(43) Date of publication of application: 22.06.2022
(73) Proprietor: Microport Navibot (Suzhou) Co., Ltd., Suzhou, Jiangsu 215000 (CN)
(72) Inventor: SUN, Feng, Suzhou, Jiangsu 215000 (CN); HU, Fangqiu, Suzhou, Jiangsu 215000 (CN); HE, Chao, Suzhou, Jiangsu 215000 (CN); LI, Tao, Suzhou, Jiangsu 215000 (CN); LIU, Pengfei, Suzhou, Jiangsu 215000 (CN)
(74) Representative: Patentship Patentanwaltsgesellschaft mbH
(86) International application number: PCT/CN2020/095217
(87) International publication number: WO 2021/063023

(56) References cited:
- WO-A1-2018/103945
- CN-A- 105 813 592
- CN-A- 107 405 170
- CN-A- 110 114 031
- CN-A- 110 711 029
- US-A1- 2016 249 986
- US-A1- 2018 085 135

## Description

### TECHNICAL FIELD

The present invention relates to robot-aided surgery systems, and more particularly to an osteotomy guide and an orthopedic surgery system.

### BACKGROUND

In the procedure of artificial joint replacement, various locators, guides and the like may be used to achieve the accuracy of the osteotomy procedure prior to replacement with an artificial joint. Many methods have been proposed for helping a surgeon position an osteotomy guide during a total knee replacement (TKR) procedure.

However, existing devices/methods for positioning an osteotomy guide during a surgical procedure are associated with insufficient positioning accuracy and other drawbacks. Therefore, it is desirable to develop a method and surgery system capable of positioning an osteotomy guide with higher accuracy.

WO 2018/103945 A1 discloses a surgical system for cutting an anatomical structure of a patient. US 2016/249986 A1 discloses a surgical instrument.

### SUMMARY OF THE INVENTION

The present invention is set out in the appended set of claims.

In view of the above, it is an objective of the present invention to provide an osteotomy guide and an orthopedic surgery system, in which more accurate positioning of the osteotomy guide is achieved by tracking and feeding back the position and orientation of the osteotomy guide in real time and controlling its movement using a robotic arm.

According to a first aspect not covered by the present invention and shown only for illustrative purpose, there is provided a method of correcting the position of an osteotomy guide, including:
controlling a robotic arm to move based on current and desired positions of a guide fiducial marker attached to the osteotomy guide or to the robotic arm, thereby driving the osteotomy guide and the guide fiducial marker to move so that the guide fiducial marker reaches the desired position,
wherein the position of the guide fiducial marker is configured to characterize the position of the osteotomy guide.

Optionally, in the method not covered by the present invention and shown only for illustrative purpose, prior to the control step, a checking step may be performed to check whether the osteotomy guide and/or the guide fiducial marker has/have undergone deformation. The checking step may include:
based on initial positional information of a checking fiducial marker attached to the osteotomy guide relative to the guide fiducial marker before delivery from the factory, as well as on information about the current position of the checking fiducial marker relative to the guide fiducial marker during use, determining whether there is a match between the initial positional information of the checking fiducial marker and the information about the current position thereof;
if there is a match, determining that the osteotomy guide and/or the guide fiducial marker has/have not undergone deformation and performing the control step for controlling the robotic arm to move based on the current and desired positions of the guide fiducial marker attached to the osteotomy guide or to the robotic arm; and
if there is not a match, determining that the osteotomy guide and/or the guide fiducial marker has/have undergone deformation, performing a correction step for correcting the guide fiducial marker attached to the osteotomy guide or to the robotic arm; and
if there is not a match, determining that the osteotomy guide and/or the guide fiducial marker has/have undergone deformation, performing a correction step for correcting the position of the osteotomy guide relative to the guide fiducial marker, and then performing the control step for controlling the robotic arm to move based on the current and desired positions of the guide fiducial marker attached to the osteotomy guide or to the robotic arm.

Optionally, in the method not covered by the present invention and shown only for illustrative purpose, the correction step may include:
acquiring positional information of at least two correcting fiducial markers attached to the osteotomy guide relative to the guide fiducial marker; and
deriving the information about the current position of the osteotomy guide relative to the guide fiducial marker from the positional information of the at least two correcting fiducial markers relative to the guide fiducial marker and updating the position of the osteotomy guide relative to the guide fiducial marker using the derived information about the current position.

Optionally, in the method not covered by the present invention and shown only for illustrative purpose, with positional information of one of the correcting fiducial markers being denoted as *T*₁ and represented by a positional representation of the specific correcting fiducial marker in a coordinate system of the guide fiducial marker and positional information of another one of the correcting fiducial markers being denoted as *T*₂ and represented by a positional representation of this correcting fiducial marker in the coordinate system of the guide fiducial marker,
the information about the current position of the osteotomy guide relative to the guide fiducial marker may be derived by the steps of:
deriving a positional representation *T*₀ of a center of an osteotomy guide block in the coordinate system of the guide fiducial marker and a positional representation *T*₃ of a surface of the osteotomy guide block in the coordinate system of the guide fiducial marker from the positional representations *T*₁ and *T*₂ in the coordinate system of the guide fiducial marker; and
deriving the position and orientation of the osteotomy guide relative to the guide fiducial marker from both the positional representation *T*₀ of the center of the osteotomy guide block in the coordinate system of the guide fiducial marker and the positional representation *T*₃ of the surface of the osteotomy guide block in the coordinate system of the guide fiducial marker.

Optionally, prior to the control step, the method not covered by the present invention and shown only for illustrative purpose may include the step of
deriving a desired travel path for the guide fiducial marker from the current and desired positions thereof,
wherein in the control step, the robotic arm is controlled to move based on the current position of the guide fiducial marker so that the guide fiducial marker follows the desired travel path to move to the desired position.

Optionally, in the method not covered by the present invention and shown only for illustrative purpose, the desired position for the guide fiducial marker may be determined from both a positional and orientational mapping between the guide fiducial marker and the osteotomy guide and a target position for the osteotomy guide.

Optionally, in the method not covered by the present invention and shown only for illustrative purpose, the positional and orientational mapping between the guide fiducial marker and the osteotomy guide may include positional and orientational mappings between a number of guide features on the osteotomy guide and the guide fiducial marker.

Optionally, in the method not covered by the present invention and shown only for illustrative purpose, the positional and orientational mapping between each of the guide features and the guide fiducial marker may be determined by:
determining a relative positional relationship among fiducial marker balls on the guide fiducial marker and establishing a coordinate system of the guide fiducial marker based on the relative positional relationship;
determining positional information of a center of an osteotomy guide block in the coordinate system of the guide fiducial marker; and
determining the position and orientation of the guide feature in the coordinate system of the guide fiducial marker from both positional information of the guide feature relative to the center of the osteotomy guide block and the positional information of the center of the osteotomy guide block in the coordinate system of the guide fiducial marker.

In a second aspect not covered by the present invention and shown only for illustrative purpose, there is provided a computer readable storage medium storing thereon instructions, which when executed by a processor, perform the method as defined in any of the preceding paragraphs.

In a third aspect of the present invention, there is provided an orthopedic surgery system, including a control device, a navigation device, a robotic arm and an osteotomy guide, the osteotomy guide attached to one end of the robotic arm, the robotic arm configured to adjust the position and orientation of the osteotomy guide,
the navigation device including a tracker and a guide fiducial marker, the guide fiducial marker attached to the osteotomy guide or the robotic arm, the tracker configured to track the current position of the guide fiducial marker and generate information about the current position, the position of the guide fiducial marker configured to characterize the position of the osteotomy guide,
the control device configured to control the robotic arm to move based both on the information about the current position of the guide fiducial marker fed back from the tracker and on information about a desired position for the guide fiducial marker, thereby driving the osteotomy guide and the guide fiducial marker to move so that the guide fiducial marker reaches the desired position.

Optionally, the orthopedic surgery system may further include a checking device configured to check whether the osteotomy guide and/or the guide fiducial marker has/have undergone deformation, wherein:
the osteotomy guide includes an osteotomy guide block provided thereon with a plurality of guide features for providing osteotomy guidance, and the checking device includes at least one checking fiducial marker configured for detachable attachment to the osteotomy guide block;
the tracker is configured to record initial positional information of the checking fiducial marker relative to the guide fiducial marker before delivery from the factory and record information about the current position of the checking fiducial marker relative to the guide fiducial marker after each surgical use; and
the control device is configured to determine whether there is a match between the initial positional information of the checking fiducial marker and the information about the current position of the checking fiducial marker, if there is a match, determine that the osteotomy guide and/or the guide fiducial marker has/have not undergone deformation, and if there is not a match, determine that the osteotomy guide and/or the guide fiducial marker has/have undergone deformation.

Optionally, the orthopedic surgery system may further include a correcting device for correcting the position of the osteotomy guide relative to the guide fiducial marker when the checking device determines that the osteotomy guide and/or the guide fiducial marker has/have not undergone deformation, the correcting device including at least two correcting fiducial markers each configured for detachable attachment to the osteotomy guide block, wherein:
the tracker is configured to record positional information of the at least two correcting fiducial markers relative to the guide fiducial marker, and
the control device is configured to derive the information about the current position of the osteotomy guide relative to the guide fiducial marker from the positional information of the at least two correcting fiducial markers relative to the guide fiducial marker and update the position of the osteotomy guide relative to the guide fiducial marker using the derived information about the current position.

Optionally, in the orthopedic surgery system, with positional information of one of the correcting fiducial markers being denoted as *T*₁ and represented by a positional representation of the specific correcting fiducial marker in a coordinate system of the guide fiducial marker and positional information of another one of the correcting fiducial markers being denoted as *T*₂ and represented by a positional representation of this correcting fiducial marker in the coordinate system of the guide fiducial marker,
the control device may be configured to derive a positional representation *T*₀ of a center of an osteotomy guide block in the coordinate system of the guide fiducial marker and a positional representation *T*₃ of a surface of the osteotomy guide block in the coordinate system of the guide fiducial marker from the positional representations *T*₁ and *T*₂ in the coordinate system of the guide fiducial marker and derive the position and orientation of the osteotomy guide relative to the guide fiducial marker from both the positional representation *T*₀ of the center of the osteotomy guide block in the coordinate system of the guide fiducial marker and the positional representation *T*₃ of the surface of the osteotomy guide block in the coordinate system of the guide fiducial marker.

Optionally, in the orthopedic surgery system, the navigation device may further include a base fiducial marker which is maintained stationary, and the position of the guide fiducial marker may be relative to that of the base fiducial marker.

Optionally, in the orthopedic surgery system, the control device may be configured to provide a desired travel path defined by a plurality of positional points and to control the robotic arm to move so that the guide fiducial marker follows the desired travel path to reach the desired position.

Optionally, the orthopedic surgery system may further include a storage device for storing a positional and orientational mapping between the guide fiducial marker and the osteotomy guide.

Optionally, in the orthopedic surgery system, the osteotomy guide may include an osteotomy guide block provided thereon with a number of guide features for providing multiple osteotomy guidance modes, wherein the positional and orientational mapping between the guide fiducial marker and the osteotomy guide includes positional and orientational mappings between the guide features and the guide fiducial marker.

Optionally, in the orthopedic surgery system, the positional and orientational mapping between each of the guide features and the guide fiducial marker may be determined by:
determining a relative positional relationship among fiducial marker balls on the guide fiducial marker and establishing a coordinate system of the guide fiducial marker based on the relative positional relationship;
determining positional information of a center of an osteotomy guide block in the coordinate system of the guide fiducial marker; and
determining the position and orientation of the guide feature in the coordinate system of the guide fiducial marker from both positional information of the guide feature relative to the center of the osteotomy guide block and the positional information of the center of the osteotomy guide block in the coordinate system of the guide fiducial marker.

Optionally, in the orthopedic surgery system, the osteotomy guide block may be provided therein with at least one checking hole to which the respective checking fiducial marker(s) is/are secured, wherein each checking fiducial marker has a step with a step surface that is parallel to a surface of the osteotomy guide block.

Optionally, in the orthopedic surgery system, the osteotomy guide block may have an upper surface in which the guide features are provided, wherein the checking hole is provided in the upper surface so as to extend perpendicularly thereto, and wherein the step surface is matched with the upper surface.

Optionally, in the orthopedic surgery system, an axis line of the checking hole may be located in a plane of symmetry of the osteotomy guide block, wherein an end face of the checking hole, the upper surface of the osteotomy guide block and the step surface are co-planar.

Optionally, in the orthopedic surgery system, the positional information of the checking fiducial marker relative to the guide fiducial marker may include the position and orientation of a front end point of the checking fiducial marker in a coordinate system of the guide fiducial marker.

Optionally, in the orthopedic surgery system, the position of each correcting fiducial marker in the coordinate system of the guide fiducial marker may include:
the position and orientation of a front end point of the correcting fiducial marker in the coordinate system of the guide fiducial marker; and
the position and orientation of a step surface of the correcting fiducial marker in the coordinate system of the guide fiducial marker,
wherein the step surface is of a step of the correcting fiducial marker and is parallel to a surface of the osteotomy guide block.

Optionally, in the orthopedic surgery system, the osteotomy guide block may be provided therein with correcting hole(s), wherein two correcting fiducial markers are secured to the respective correcting holes, or to one checking hole and one correcting hole.

Optionally, in the orthopedic surgery system, each correcting hole may have an end face that is co-planar with both the surface of the osteotomy guide block and a step surface of each correcting fiducial marker.

Optionally, in the orthopedic surgery system, the osteotomy guide may include an osteotomy guide block and a mounting interface, the mounting interface connected to the osteotomy guide block by a connecting shaft, the osteotomy guide block provided thereon with a plurality of guide features for providing osteotomy guidance, the mounting interface attached detachably to the end of the robotic arm.

Optionally, in the orthopedic surgery system, the connecting shaft may be an eccentric crank that is connected to both the osteotomy guide block and the mounting interface so that an axis of rotation of the mounting interface is offset from an axis of rotation of the osteotomy guide block.

Optionally, in the orthopedic surgery system, the osteotomy guide may further include a holder, wherein the osteotomy guide block, the eccentric crank, the holder and the mounting interface are sequentially connected together in this order, and wherein the holder is provided therein with fiducial marker mounting holes.

Optionally, in the orthopedic surgery system, the osteotomy guide block may be attached detachably to the eccentric crank.

Optionally, in the orthopedic surgery system, the guide features may include a number of guide slots provided on a single surface of the osteotomy guide block, or on different surface around an axis line of the osteotomy guide block.

Optionally, in the orthopedic surgery system, the guide slots may be 0° guide slots or 45° guide slots.

Optionally, in the orthopedic surgery system, the osteotomy guide may include two osteotomy guide blocks mirrored to each other and each provided thereon with a mounting interface configured for detachable connection with the eccentric crank.

Optionally, in the orthopedic surgery system, the osteotomy guide may include one osteotomy guide block provided thereon with two mounting interfaces arranged in opposition to each other, wherein the eccentric crank is selectively connected detachably to one of the mounting interfaces.

Optionally, the orthopedic surgery system may further include a sterile bag disposed over the end of the robotic arm so that an end portion of the sterile bag covers a connecting port of the robotic arm, wherein the connecting port is connected detachably to the osteotomy guide.

Optionally, in the orthopedic surgery system, the sterile bag may be provided in one end thereof with passage holes.

According to a further aspect of the present invention, there is provided an osteotomy guide for use in the orthopedic surgery system as defined above. The osteotomy guide includes an osteotomy guide block, a connecting shaft and a mounting interface. The osteotomy guide block is provided thereon with a number of guide features for providing multiple osteotomy guidance modes, and the connecting shaft is connected to both the osteotomy guide block and the mounting interface at its opposing ends. The mounting interface is configured for detachable connection with one end of a robotic arm.

Optionally, in the osteotomy guide, the guide features may include guide slot(s) and guide hole(s).

Optionally, in the osteotomy guide, the connecting shaft may be an eccentric crank.

Optionally, the osteotomy guide may further include a holder, wherein the osteotomy guide block, the eccentric crank, the holder and the mounting interface are sequentially connected together in this order, and wherein the holder is provided therein with fiducial marker mounting holes.

Optionally, in the osteotomy guide, the osteotomy guide block may be detachably attached to the eccentric crank.

Optionally, in the osteotomy guide, each guide slot may extend through a surface of the osteotomy guide block and thus have end openings.

Optionally, in the osteotomy guide, each guide slot may be flared in shape.

Optionally, in the osteotomy guide, a plurality of guide slots may be included and arranged in different surfaces around an axis line of the osteotomy guide block. Optionally, each guide slot may be a 0° guide slot or a 45° guide slot.

Optionally, in the osteotomy guide, the guide slot(s) may include at least one of 0° guide slot(s), 45° guide slot(s) and trochlear cutting guide slot(s), and the guide hole(s) may include at least one of guide hole(s) for the mounting of a femoral prosthesis, guide hole(s) for the locating of left tibial tooling, guide hole(s) for the locating of right tibial tooling and securing hole(s) for the osteotomy guide.

Optionally, in the osteotomy guide, the guide slot(s) may include two 0° guide slots, two 45° guide slots and two trochlear cutting slots.

Optionally, in the osteotomy guide, the osteotomy guide block may be an axisymmetric structure.

Optionally, in the osteotomy guide, the osteotomy guide block may be provided thereon with two mounting interfaces arranged in opposition to each other, wherein the eccentric crank is selectively connected detachably to one of the mounting interfaces.

Optionally, in the osteotomy guide, the osteotomy guide block may be provided therein with a checking hole.

Optionally, in the osteotomy guide, the osteotomy guide block may be provided therein with a correcting hole.

In the above method, the position and orientation of the osteotomy guide are tracked and fed back in real time and are taken as a basis for controlling the robotic arm to move. In this way, the osteotomy guide can be placed and positioned more accurately without taking into account the absolute positional accuracy of the robotic arm or relying on the surgeon's experience. Moreover, in the above orthopedic surgery system, the osteotomy guide is hung on the robotic arm rather than fixed on the patient's body, avoiding causing secondary damage thereto.

Further, in the above method, through using the checking fiducial marker, it can be determined in a timely manner whether the osteotomy guide and/or guide fiducial marker has/have undergone deformation. This facilitates the replacement or correction of the osteotomy guide and/or guide fiducial marker that has/have undergone deformation, thus reducing surgical risk and improving surgical accuracy. For example, in the above method, at least two correcting fiducial markers may be further used to correct and update the position of the osteotomy guide relative to the guide fiducial marker, and such correction can be accomplished in a simple and convenient manner.

The osteotomy guide block in the above osteotomy guide may be provided with a number of guide features, which may be any of various combinations of guide holes and guide slots. These guide features can provide various osteotomy guidance modes, making the single osteotomy guide applicable to different bone cutting and drilling operations and thus eliminating the need to frequently change over from one osteotomy guide to another. This can greatly shorten the surgery time and improve surgical efficiency.

Preferably, the above osteotomy guide is implemented as an axisymmetric structure, which enables the osteotomy guide to support osteotomies on any leg. This results in cost savings, increased simplicity of the osteotomies and further improved surgical efficiency.

In the above osteotomy guide, each guide slot may be, for example, a 45° guide slot or 0° guide slot, which extends through the surface of the osteotomy guide block at both ends and thus has end openings. This makes the osteotomy guide applicable to osteotomy guidance for prostheses of various models.

In the above osteotomy guide, each guide slot may be, for example, a 45° guide slot or 0° guide slot, which is flared in shape. This also makes the osteotomy guide applicable to osteotomy guidance for prostheses of various models.

In the above osteotomy guide, guide slots (e.g., 0° or 45°) are preferably provided on different surfaces around the axis line of the osteotomy guide block. This allows the osteotomy guide to travel a reduced distance to switch to another bone surface to be cut, avoiding positional and orientational identification errors or failures that may be caused by large rotational strokes of the fiducial markers, as well as significant rotational errors of the robotic arm that may be caused by excessive positional and orientational changes, resulting in additional increases in positioning accuracy.

The axis of rotation of the above osteotomy guide is offset from that of the end section of the robotic arm. In this way, an angular adjustment of the osteotomy guide can be accomplished simply by causing the robotic arm to translate a small distance and then rotate. This can reduce rotational errors of the robotic arm, resulting in a further increase in positioning accuracy.

### BRIEF DESCRIPTION OF THE DRAWINGS

Features, characteristics and advantages of embodiments of the present invention and examples thereof will be described with reference to the accompanying drawings, in which:
Fig. 1 schematically illustrates an orthopedic surgery system being used in knee joint replacement according to an embodiment of the present invention;
Fig. 2 is a structural schematic of an osteotomy guide according to an embodiment of the present invention;
Fig. 3 schematically illustrates how the osteotomy guide translates to enable osteotomy guidance at multiple locations and orientations according to an embodiment of the present invention;
Fig. 4 schematically illustrates how the osteotomy guide rotates to shift from one bone surface to be cut to another according to an embodiment of the present invention;
Fig. 5 is a cross-sectional view of the osteotomy guide of Fig. 2 along line A-A, showing a 0° guide slot flared in shape;
Fig. 6 schematically illustrates how a checking fiducial marker checks whether the osteotomy guide and/or a guide fiducial marker has/have deformed during use according to an embodiment of the present invention;
Fig. 7 is a structural schematic of the osteotomy guide according to an embodiment of the present invention, showing correcting holes provided therein;
Fig. 8 is a diagram illustrating how the osteotomy guide is corrected using correcting fiducial markers according to an embodiment of the present invention;
Fig. 9 is a schematic illustration of the guide fiducial marker that is alternatively attached to an outer casing of an end section of a robotic arm according to an embodiment of the present invention;
Fig. 10 is a structural schematic of two osteotomy guides mirrored to each other according to an alternative embodiment of the present invention;
Fig. 11 is a schematic illustration of an alternative osteotomy guide block provided with two quick-change interfaces, which is capable of osteotomy guidance for left or right leg, according to an alternative embodiment of the present invention;
Fig. 12 is a schematic illustration of a travel path for the guide fiducial marker according to an embodiment of the present invention;
Fig. 13 schematically illustrates guidance for tibial plateau preparation according to an embodiment of the present invention;
Fig. 14 schematically illustrates guidance for anterior femoral cutting according to an embodiment of the present invention;
Fig. 15 shows a structural schematic of a first example of the osteotomy guide, which includes only one 0° guide slot;
Fig. 16 shows a structural schematic of a second example of the osteotomy guide, which includes two 0° guide slots;
Fig. 17 shows a structural schematic of a third example of the osteotomy guide, which includes two trochlear cutting slots and two 0° guide slots;
Fig. 18 shows a structural schematic of a fourth example of the osteotomy guide, which includes one 0° guide slot and two guide holes;
Fig. 19 shows a structural schematic of a fifth example of the osteotomy guide, which includes two 0° guide slots and two guide holes;
Fig. 20 shows a structural schematic of a sixth example of the osteotomy guide, which includes two 0° guide slots, two trochlear cutting slots and two guide holes;
Fig. 21 shows a structural schematic of a seventh example of the osteotomy guide, which includes two securing holes and multiple guide slots;
Fig. 22 shows a structural schematic of an eighth example of the osteotomy guide, which includes a rectangular trochlear cutting notch, an elongate guide slot and two guide holes;
Fig. 23 shows a structural schematic of a ninth example of the osteotomy guide, which includes guide slots each having an end opening at one end;
Fig. 24 shows a structural schematic of a tenth example of the osteotomy guide, which is a tibial tooling guide;
Fig. 25 shows a structural schematic of an eleventh example of the osteotomy guide, which is a trochlear cutting guide;
Fig. 26a shows a structural schematic of a sterile bag disposed over the end section of the robotic arm according to an embodiment of the present invention; and
Fig. 26b shows a partially enlarged view of the sterile bag that is provided therein with passage holes at one end according to an embodiment of the present invention.

In these figures,
1-surgical cart; 2-robotic arm; 3-guide fiducial marker; 4-osteotomy guide; 5-surgical instrument; 6-tracker; 7-slave monitor; 8-master monitor; 9-navigation cart; 10-keyboard; 11-femoral fiducial marker; 12-femur; 13-tibial fiducial marker; 14-tibia; 15-base fiducial marker; 16-checking fiducial marker; 17-patient; 18-surgeon;
40, 41, 42, 43, 20, 21, 22, 23, 24-osteotomy guide blocks;
401-mounting interface; 402-holder; 4021-fiducial marker mounting hole; 403-eccentric crank; 404-guide hole for the mounting of a femoral prosthesis; 405-guide slot for right trochlear cutting; 406-guide hole for the locating of left tibial tooling; 407-first 0° guide slot; 408-first 45° guide slot; 409-checking hole; 410-second 45° guide slot; 411-second 0° guide slot; 412-guide slot for left trochlear cutting; 413-guide hole for the locating of right tibial tooling; 414a-first correcting hole; 414b-second correcting hole;
415, 421, 431, 432-quick change interfaces; 201, 211-0° guide slot; 221-trochlear cutting guide slot; 231, 241, 251, 271-guide holes; 261-locating hole; 272-rectangular notch; 273-elongate guide slot; 281-end opening; 31-sterile bag; 311-passage hole; 312-fastener.

### DETAILED DESCRIPTION

The techniques disclosed in embodiments hereof will be described clearly and fully hereunder in conjunction with the appended drawings. Apparently, the embodiments set forth herein are merely some, but not all, of the possible embodiments of this invention. Any and all other embodiments devisable by skilled artisans in light of the disclosed embodiments without paying any creative effort are considered to fall within the scope of the invention.

As used herein, the singular forms "a", "an" and "the" include plural referents, unless the context clearly dictates otherwise. As used herein, the term "or" is employed in the sense including "and/or", unless the context clearly dictates otherwise. As used herein, the term "several" is used in the sense of "at least one", unless the context clearly dictates otherwise. As used herein, the term "at least two" is used usually in the sense of "two or more", unless the context clearly dictates otherwise. Moreover, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items.

The present invention discloses a method of correcting the position of an osteotomy guide, which is not covered by the present invention and is shown only for illustrative purpose, based on the idea that the position of the osteotomy guide is tracked using a guide fiducial marker that is attached to the osteotomy guide or to a robotic arm. Positional information of the guide fiducial marker (indicating a current position and a desired position thereof) is acquired and taken as a basis for controlling the robotic arm to move. As a result, the osteotomy guide that is attached to one end of the robotic arm, as well as the guide fiducial marker, moves so that the guide fiducial marker reaches the desired position. Since the position of the guide fiducial marker is mapped to the position of the osteotomy guide, any adjustment in the position or orientation of the guide fiducial marker results in an adjustment in the position and orientation of the osteotomy guide. That is, the position of the osteotomy guide is characterized by the position of the guide fiducial marker. This dispenses with the needs for taking into account absolute positional accuracy of the robotic arm and for relying on the surgeon's experience and allows the osteotomy guide to be placed more accurately, resulting in improved osteotomy guide positioning accuracy and increased surgical accuracy.

In order for accurate positioning of an osteotomy guide to be achieved, the present invention also provides an orthopedic surgery system including a control device, a navigation device, a robotic arm and the osteotomy guide (or bone cutting guide). The osteotomy guide or the bone cutting guide is attached to an end of the robotic arm that is configured to adjust the position and orientation of the osteotomy guide. The navigation device includes a tracker and a guide fiducial marker. The guide fiducial marker is attached to the osteotomy guide or to the robotic arm, and the tracker is used to track the current position of the guide fiducial marker and generate information about the current position. In practical use, based on information about the current and desired positions of the guide fiducial marker fed back from the tracker, the control device can control the robotic arm to move, thus driving the osteotomy guide and the guide fiducial marker to move so that the guide fiducial marker reaches the desired position. In addition to the above advantages, the orthopedic surgery system of the present invention allows the osteotomy guide to be hung on the robotic arm rather than fixed on a patient's body, avoiding causing secondary damage to the patient's body.

However, the present invention is not limited to any particular application where the orthopedic surgery system can be used. For example, in addition to knee joint replacement, the orthopedic surgery system may also be used in other orthopedic surgeries. The following description is set forth in the context of the orthopedic surgery system being used in knee j oint replacement as an example, but this should not be construed as limiting the scope of the present invention in any sense.

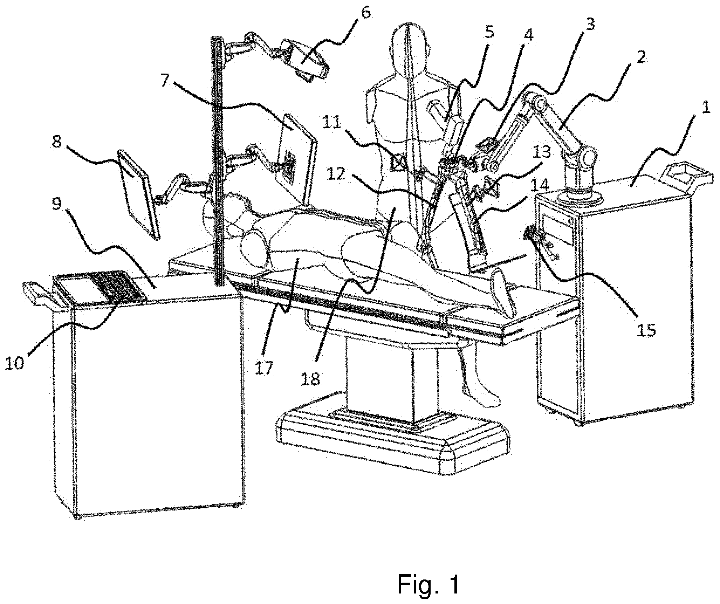

Fig. 1 schematically illustrates an orthopedic surgery system being used in knee joint replacement according to an embodiment of the present invention. As shown in Fig. 1, the orthopedic surgery system includes a control device, a navigation device, a robotic arm 2 and an osteotomy guide 4. The control device may be practically implemented as a computer equipped with a controller, a master monitor 8, a keyboard 10 and, more preferably, a slave monitor 7. In this embodiment, the slave monitor 7 and the master monitor 8 display the same content, which is, for example, an image of a location where an osteotomy is being performed. The navigation device may be a navigator/sensor based on electromagnetic or optical positioning. In some embodiments, the navigation device may be based on optical positioning, which provides higher measurement accuracy and allows increased osteotomy guide positioning accuracy, compared to other navigation techniques.

The following description is set forth in the context of the navigation device being based on optical positioning as an example, but this should not be construed as limiting in any way.

In a specific implementation, the navigation device includes a navigation markers and a tracker 6. The navigation markers include a base fiducial marker 15 and a guide fiducial marker 3. The base fiducial marker 15 is immobilized, for example, the base fiducial marker 15 is fixed on a surgical cart 1 so as to provide a base coordinate system (or base fiducial marker coordinate system), while the guide fiducial marker 3 is attached to the osteotomy guide 4 to allow tracking of the position of the osteotomy guide 4. The osteotomy guide 4 is attached to and supported at an end of the robotic arm 2, thereby the spatial position and orientation of the osteotomy guide 4 are adjustable.

In practice, the tracker 6 is used to capture a signal (preferably, an optical signal) reflected from the guide fiducial marker 3 and record a position of the guide fiducial marker 3 (i.e., its position and orientation in the base coordinate system). The controller then controls, based on instructions stored in a storage device and the current and desired positions of the guide fiducial marker, the robotic arm 2 to move, thus driving the osteotomy guide 4 and the guide fiducial marker 3 to move, so that the guide fiducial marker 3 reaches and stays at its desired position that is mapped to a desired position of the osteotomy guide 4.

Thus, in applications of the orthopedic surgery system, the osteotomy guide 4 can be automatically located. Moreover, during surgery, the guide fiducial marker 3 tracks the position and orientation of the osteotomy guide 4 and feeds information thereabout in real time, based on which, the robotic arm is controlled to moved, resulting in an adjustment in the position and orientation of the osteotomy guide. In this way, in addition to high positioning accuracy of the osteotomy guide being achievable, the osteotomy guide 4 can be supported on the robotic arm 2 rather than fixed on the patient's body, avoiding causing secondary damage to the patient's body.

In general, the orthopedic surgery system further includes a surgical cart 1 and a navigation cart 9. The control device and part of the navigation device are mounted on the navigation cart 9. For example, the controller may be mounted inside the navigation cart 9, while the keyboard 10 may be placed outside the navigation cart 9 to facilitate manipulation. Additionally, the master monitor 8, the slave monitor 7 and the tracker 6 may be all fixed to a mast erected upright on a surface of the navigation cart 9, with the robotic arm 2 being mounted on the surgical cart 1. The use of the surgical cart 1 and the navigation cart 9 allows ease of surgical operation.

The use of the orthopedic surgery system according to this embodiment in a knee joint replacement procedure generally involves the following operations.

First of all, both the surgical cart 1 and the navigation cart 9 are moved to proper locations beside a patient's bed.

The navigation markers (which may further include a femoral fiducial marker 11 and a tibial fiducial marker 13), the osteotomy guide 4 and other necessary components (e.g., a sterile bag) are then configured to be ready for use.

After that, a surgeon 18 performs preoperative planning based on a CT/MR scan model of a region of bone of a patient 17 in need of an osteotomy treatment that has been imported to the aforesaid computer to develop an osteotomy plan which includes, for example, information about coordinates of a bone surface to be cut, the prosthesis' model, target position and orientation and the like. Specifically, image data of the patient's knee joint may be obtained from a CT/MR scan and used to create a three-dimensional (3D) digital knee joint model for developing an osteotomy plan, which may serve as a basis for the surgeon to carry out preoperative assessment. More specifically, the osteotomy plan may be developed based on the 3D digital knee joint model, as well as on dimensions of the prosthesis, a target location for an osteotomy plate and the like, and may be output in the form of a surgical report, which may specify a series of reference data including the coordinates of the bone surface to be cut, an amount of bone to be cut away, an osteotomy angle, the size of the prosthesis, the target location for the prosthesis, auxiliary surgical instruments, etc. In particular, it may contain a series of passages of text for the surgeon's reference, which may specify the reason for osteotomy angle, for example. The 3D digital knee joint model may be displayed on the master monitor 8. During the preoperative planning, the surgeon may input surgical parameters through the keyboard 10.

Subsequent to the preoperative assessment, the surgeon 18 registers feature points of the patient's femur and tibia (including multiple anatomically characteristic points of the patient's femur and multiple anatomically characteristic points of the patient's tibia) with a pointer or a pod equipped with a tracking feature, and uses the navigation device to record positions of all the feature points of the patient's tibia 14 and femur 12 relative to the base fiducial marker 15 that is taken as a reference. Information about the positions of all the feature points is then sent to the controller, which then determines the actual positions and orientations of the femur 12 and the tibia 14 using a feature matching algorithm and correlates them to their respective graphic representations in the CT/MR image.

Afterward, the navigation device correlates the actual positions and orientations of the femur and the tibia respectively to the fiducial markers fixed to the femur and the tibia, allowing the femoral fiducial marker 11 and tibial fiducial marker 13 to track the current positions of the bones in real time. Moreover, during surgery, as long as the fiducial markers are fixed in position relative to the respective bones, any movement of the bones will not lead to an adverse effect on the surgical outcome.

Thereafter, the navigation device sends the coordinates of the bone surface to be cut that have been determined during the preoperative planning to the robotic arm 2, the robotic arm 2 then locates the bone surface to be cut with the aid of the guide fiducial marker 3 and moves to a proper location. After causing the robotic arm 2 to remain stationary (i.e., in an immobilized state), the surgeon performs osteotomy guidance and/or drilling operations with the aid of the osteotomy guide 4 using surgical instrument 5 such as a swing saw or an electric drill. Following the completion of the osteotomy guidance and/or drilling operations, the surgeon may set the prosthesis in place and carry out other necessary operations.

In this embodiment, the navigation markers further include the femoral fiducial marker 11 and the tibial fiducial marker 13. The femoral fiducial marker 11 is used to locate/track the spatial position and orientation of the femur 12, while the tibial fiducial marker 13 is used to locate/track the spatial position and orientation of the tibia 14. Although the guide fiducial marker 3 has been described above as being attached to the osteotomy guide 4, in alternative embodiment, the guide fiducial marker 3 may also be attached to an end section of the robotic arm 2.

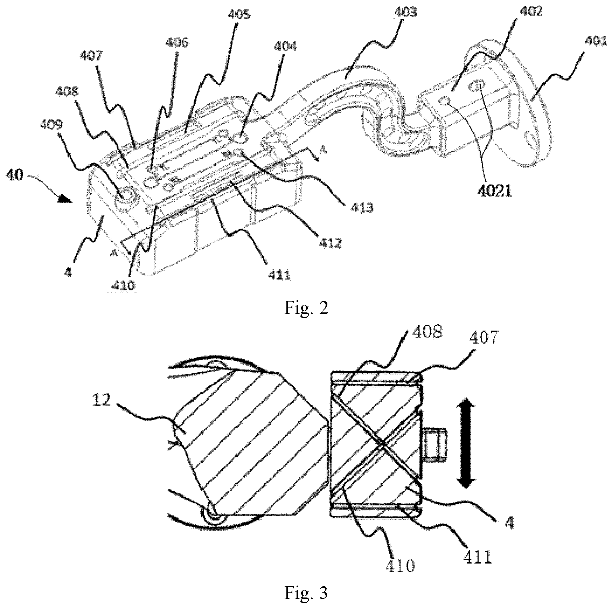

Fig. 2 shows a structural schematic of an osteotomy guide according to an embodiment of the present invention. As shown in Fig. 2, the osteotomy guide 4 according to the illustrated embodiment is intended for use in knee joint replacement and includes an osteotomy guide block 40 with guide features provided in the osteotomy guide block 40, the guide features may include guide slots, guide holes, or both. That is, the guide features on the osteotomy guide block 40 may be either guide slots or guide holes, or a combination thereof, thus supporting the guidance of one or more osteotomies that may be involved in knee joint replacement. Particular examples of such osteotomies may include distal, anterior and posterior femoral cutting, anterior and posterior femoral oblique cutting, trochlear cutting, drilling of holes for femoral prosthesis fixation studs, tibial plateau preparation and drilling of locating holes for tibial keel preparation tools. In this way, the single osteotomy guide can handle various osteotomy guidance and drilling operations without the need to change over to another osteotomy guide, thus resulting in a significantly shortened surgery time and improved surgical efficiency.

As mentioned earlier, the position of the osteotomy guide is characterized by the position of the guide fiducial marker. To this end, it is necessary to calibrate in advance a positional and orientational mapping (correlation) between the guide fiducial marker 3 and the osteotomy guide 4. In this embodiment, the positional and orientational mapping between the guide fiducial marker 3 and the osteotomy guide 4 includes a positional and orientational mapping between all the guide features and the guide fiducial marker 3. For example, if the guide features include both guide slots and guide holes, the positional and orientational mapping between the guide fiducial marker 3 and the osteotomy guide 4 may include information about the position of each guide slot of the osteotomy guide 4 in the guide fiducial marker coordinate system and information about the position of each guide hole of the osteotomy guide 4 in the guide fiducial marker coordinate system.

In this embodiment, the positional and orientational mapping of each guide feature (e.g., guide slot or guide hole) to the guide fiducial marker 3 may be determined by the steps as follows:
Step 1: Determine a relative positional relationship between fiducial marker balls (generally four) in the guide fiducial marker 3 and establish the guide fiducial marker coordinate system based on the relative positional relationship.
Step 2: Determine coordinates (i.e., positional information) of a center (geometric center or centroid) of the osteotomy guide block 40 in the guide fiducial marker coordinate system.
Step 3: Determine positional information (including both positional and orientational information) of the guide features in the guide fiducial marker coordinate system as their positional and orientational mapping to the guide fiducial marker 3 from both the positional information of the guide features relative to the center of the osteotomy guide block 40 and the coordinates (or positional information) of the center of the osteotomy guide block 40 in the guide fiducial marker coordinate system.

These pre-calibrated positions and orientations of all the guide features of the osteotomy guide block in the guide fiducial marker coordinate system may be recorded and subsequently retrieved by the controller for position transformation calculations. Preferably, the above steps for determining the positional and orientational mapping of each guide feature to the guide fiducial marker are performed by the controller.

With continued reference to Fig. 2, the osteotomy guide 4 further includes a mounting interface 401, the mounting interface 401 is connected to the osteotomy guide block 40 by a connecting shaft and is detachably connected to one end of the robotic arm 2, thus attaching the osteotomy guide 4 to the robotic arm. The mounting interface 401 may be a flange disc. In this case, it may be secured to a flange arranged at the end of the robotic arm by fasteners such as screws, locating pin, etc. In alternative embodiments, in order to allow quick attachment and detachment, the mounting interface 401 may be a quick-change interface, which can establish a quick-disconnect connection with the end of the robotic arm 2.

The osteotomy guide 4 further includes the connecting shaft that is connected at its opposite ends respectively to the osteotomy guide block 40 and the mounting interface 401. As shown in Fig. 2, the connecting shaft is preferred to be an eccentric crank 403, which offsets an axis of rotation of the mounting interface 401 (i.e., an axis of spin rotation of the end section of the robotic arm) from an axis of rotation of the osteotomy guide block 40. When it is needed to change an angle of the osteotomy guide 4 to allow the cutting of another bone surface, this can be simply accomplished by causing the robotic arm 2 to translate a small distance and then rotate as appreciate. This can avoid positional and orientational identification errors or failures that may be caused by large rotational strokes of the guide fiducial marker 3, as well as significant rotational errors of the robotic arm that may be caused by excessive positional and orientational changes, resulting in additional increases in positioning accuracy of the osteotomy guide. More preferably, the eccentric crank 403 is detachably coupled to the osteotomy guide block 40. This enables guidance of different osteotomies simply by replacing the osteotomy guide block instead of replacing the entire guide, resulting in cost savings. Further, the eccentric crank 403 may be connected to the osteotomy guide block 40 by a quick-change interface. The present invention is not limited to any structure of the quick-change interface.

As shown in Fig. 2, in one embodiment, the osteotomy guide 4 further includes a holder 402 for fixedly holding the guide fiducial marker 3. The osteotomy guide block 40, the eccentric crank 403, the holder 402 and the mounting interface 401 are sequentially connected together in this order. In this embodiment, the holder 402 is provided therein with fiducial marker mounting holes 4021 for mounting the guide fiducial marker 3. The number of the fiducial marker mounting holes 4021 may be, but is not limited to, two.

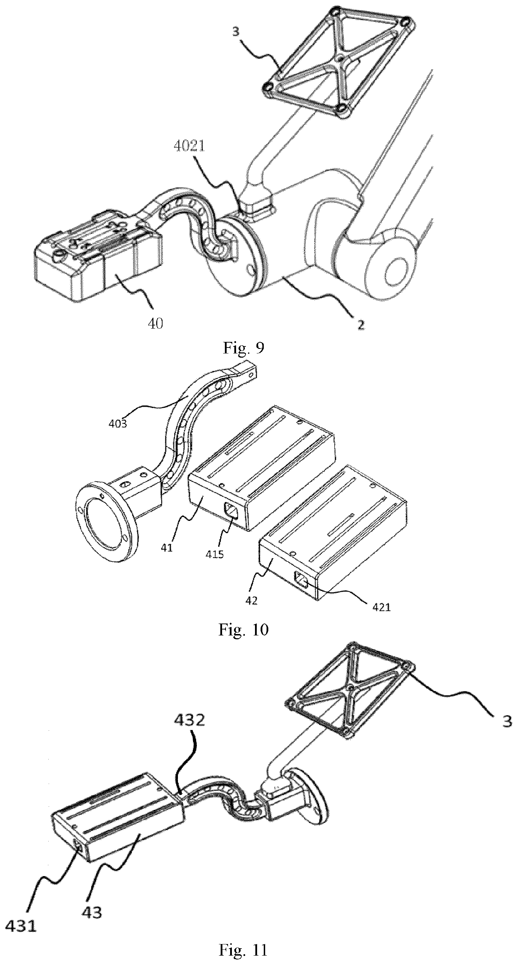

As shown in Fig. 9, in alternative embodiments, the holder 402 may be omitted. In these cases, the osteotomy guide block 40, the eccentric crank 403 and the mounting interface 401 are sequentially connected together in this order, and the guide fiducial marker 3 is directly mounted on an outer casing of the end section of the robotic arm. This can not only simplify the structure of the osteotomy guide 4, but can also bring the guide fiducial marker 3 away from the surgical area, thereby avoiding accidental touching of the fiducial marker during surgery, which may adversely affects the positioning performance. For example, the outer casing of the end section of the robotic arm 2 may be provided therein with fiducial marker mounting holes 4021 for mounting the guide fiducial marker 3.

With continued reference to Fig. 2, in order to enable the osteotomy guide 4 to guide various osteotomies, according to preferred embodiments of the present invention, the guide features of the osteotomy guide block 40 include:
guiding holes 404 for the mounting of femoral prostheses; a cutting guide slot 405 for the right trochlear groove; guide holes 406 for the locating of a tool used for the left tibia; a first 0° guide slot 407; a first 45° guide slot 408; a second 45° guide slot 410; a second 0° guide slot 411; a cutting guide slot 412 for the left trochlear groove; and guide holes 413 for the locating of a tool used for the right tibia.

In practical applications, the single osteotomy can support the osteotomy guidance and drilling operations including distal, anterior and posterior femoral cutting, anterior and posterior femoral oblique cutting, trochlear cutting, drilling of holes for femoral prosthesis fixation studs, tibial plateau preparation and drilling of locating holes for tools used in tibial keel preparation of both left and right legs simply by adjusting the position and orientation of the osteotomy guide 4 through the robotic arm 2. This allows easy and convenient osteotomies in knee joint replacement, greatly shortens the surgery time and improves surgical efficiency.

In addition, during use of the osteotomy guide 4 according to this embodiment, as shown in Fig. 3, with the 0° guide slots (407 and 411) and 45° guide slots (408 and 410) provided in the osteotomy guide block 40, the operations of osteotomy guidance including anterior and posterior femoral cutting and anterior and posterior femoral oblique cutting can be accomplished simply by causing the osteotomy guide 4 to translate (in the directions indicated by the arrows in Fig. 3). In this way, excessive positional and orientational changes of the guide fiducial marker 3 can be avoided, reducing rotational errors of the robotic arm 2 and position tracking errors of the fiducial marker and thus resulting in increased positioning accuracy.

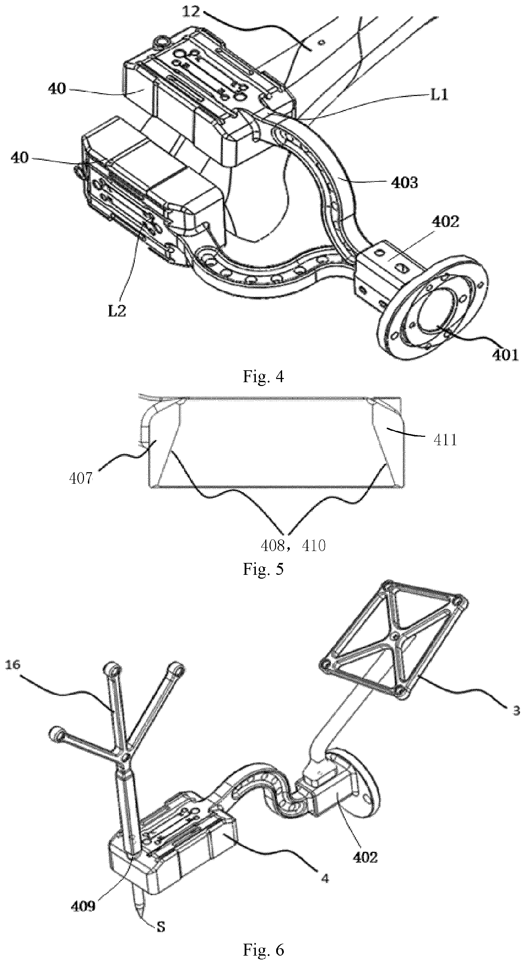

Further, in the osteotomy guide 4 according to this embodiment, as shown in Fig. 4, the eccentric crank 403 offsets a center of rotation of the osteotomy guide block 40 from a center of rotation of the mounting interface 401, and the two centers of rotation are relatively close to each other (i.e., they are slight offset from each other). With this design, when it is needed to change an angle of the osteotomy guide 4 to allow the cutting of another bone surface, this can be simply accomplished by causing the robotic arm 2 to translate a small distance and then rotate as appreciate. More specifically, when the osteotomy guide 4 is located as indicated by L1, distal cutting of the femur 12 using a surgical instrument (e.g., a swing saw) is allowed. After the osteotomy guide 4 is rotated so as to be located as indicated by L2, anterior and posterior cutting and oblique cutting of the femur 12 using appropriate surgical instruments are allowed. It is to be noted that an amount of the offset may be determined depending on factors such as the type of the prosthesis used and an allowed range of movement of the osteotomy guide, and the present invention is not limited to any particular amount of the offset.

In order to further expand the range of prostheses to which the osteotomy guide of the present invention is applicable, as shown in Fig. 5, the 0° guide slots, the 45° guide slots, or both, are preferably flared in shape. Such a shape allows a surgical instrument such as a swing saw to swing with greater amplitude in the guide slots, making the guide able to support more osteotomies required by prostheses of different models.

Preferably, the orthopedic surgery system further includes a checking device for determining a degree of deformation of the either or both of the osteotomy guide 4 and the guide fiducial marker 3. When significant deformation is identified, the osteotomy guide 4 and/or the guide fiducial marker 3 may be replaced with new one(s), or a correcting device may be employed to correct the position of the guide fiducial marker 3 relative to the osteotomy guide 4. In the latter case, the previous position and orientation of the osteotomy guide may be updated with the corrected information so that movement of the robotic arm is controlled by the corrected position and orientation of the osteotomy guide.

As shown in Fig. 6, the checking device includes at least one checking fiducial marker 16, the checking fiducial marker 16 is detachably attached to the osteotomy guide block 40. For example, at least one checking hole 409 may be provided in the osteotomy guide block 40, and the checking fiducial marker 16 may be secured to the respective checking hole 409. The checking fiducial marker 16 may define a step having a step surface extending in parallelism with a surface of the osteotomy guide block 40. In this embodiment, the step surface extends in parallelism (including coincidence) with an upper surface of the osteotomy guide block 40, and each checking hole 409 is a through hole preferably extending perpendicular to the upper surface of the osteotomy guide block 40.

In order to allow for easier calculation, most or all of the guide features (e.g., guide slots and guide holes) may be provided in the upper surface of the osteotomy guide block 40. Additionally, an axis line of the checking hole 409 may be located in a plane of symmetry of the osteotomy guide block 40, and an end face of each checking hole 409, the upper surface of the osteotomy guide block and the step surface may be co-planer. This allows for even easier calculation.

In a particular implementation, a checking process may involve, before delivery from the factory, recording initial positional information of the checking fiducial marker 16 (having been attached to the osteotomy guide block) relative to the guide fiducial marker 3 by the tracker 6. After each surgical use, the tracker 6 may record the current positional information of the checking fiducial marker 16 relative to the guide fiducial marker 3, and the controller may compare the current positional information with the initial positional information to determine whether there is a match between them. If so, the controller may determine that neither the osteotomy guide 4 nor guide fiducial marker 3 has undergone deformation. Otherwise, the controller may determine that either or both of the osteotomy guide 4 and the guide fiducial marker 3 has/have deformed.

In this embodiment, the positional information of the checking fiducial marker 16 relative to the guide fiducial marker 3 includes the position and orientation of a front end point of the checking fiducial marker in the guide fiducial marker coordinate system. As shown in Fig. 6, the front end point of the checking fiducial marker 16 may be provided by the tip S of the checking fiducial marker 16. During use, if the controller finds that the current positional information of the front end point of the checking fiducial marker is not matched with the initial information recorded before delivery from the factory, it may determine that either or both of the osteotomy guide 4 and the guide fiducial marker 3 has/have deformed. This is because if any of the guide fiducial marker and the osteotomy guide 4 undergoes deformation, it will ultimately affect the checking hole 409, and any deformation of the checking hole 409 can be identified by the checking fiducial marker.

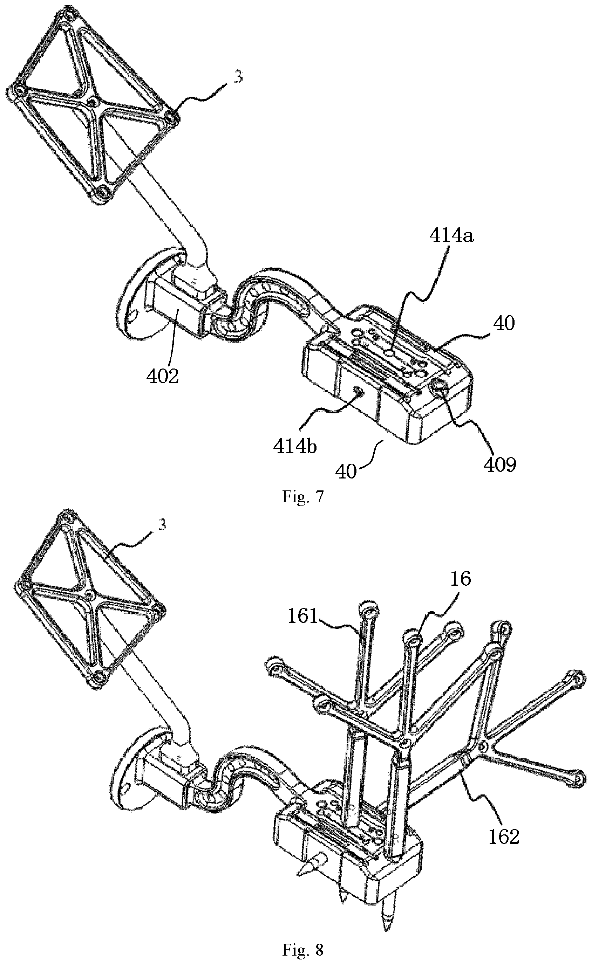

Preferably, the orthopedic surgery system further includes the correcting device. When the checking device identifies deformation of at least one of the osteotomy guide 4 and the guide fiducial marker 3, the correcting device may be used to correct the position of the osteotomy guide 4 relative to the guide fiducial marker 3 and then update the positional and orientational mapping between the osteotomy guide 4 and the guide fiducial marker 3 based on the corrected information. As shown in Figs. 7 and 8, the correcting device may include at least two correcting fiducial markers detachably attached to the osteotomy guide block 40. One or more of the correcting fiducial markers may be selected as the checking fiducial marker(s) 16. In this embodiment, two correcting fiducial markers 161 and 162 are provided to correct the position of the osteotomy guide 4 relative to the guide fiducial marker 3.

More specifically, a correction process may involve recording positional information of the two correcting fiducial markers 161 and 162 relative to the guide fiducial marker 3 by the tracker 6, followed by the controller calculating the current positional information of the osteotomy guide 4 relative to the guide fiducial marker 3 from the positional information of the two correcting fiducial markers 161 and 162 relative to the guide fiducial marker 3 and updating the positional and orientational mapping between the osteotomy guide 4 and the guide fiducial marker 3 based on the calculated information.

The positional information of the correcting fiducial marker 161 relative to the guide fiducial marker 3 may be represented by *T*₁ and the positional information of the correcting fiducial marker 162 relative to the guide fiducial marker 3 may be represented by *T*₂, wherein *T*₁ is a positional representation of the correcting fiducial marker 161 in the guide fiducial marker coordinate system, and *T*₂ is a positional representation of the correcting fiducial marker 162 in the guide fiducial marker coordinate system.

In this embodiment, a process to derive the positional information of the osteotomy guide 4 relative to the guide fiducial marker 3 includes the steps below.

At first, from the positions *T*₁ and *T*₂ of the two correcting fiducial marker 161 and 162 in the guide fiducial marker coordinate system, the controller calculates the position *T*₀ of a center of the osteotomy guide block 40 in the guide fiducial marker coordinate system and the position *T*₃ of a surface of the osteotomy guide block 40 (which is matched with the aforementioned step surface and is, for example, the upper surface) in the guide fiducial marker coordinate system.

The controller then determines the position and orientation of the osteotomy guide 4 relative to the guide fiducial marker 3 from both the position *T*₀ of the center of the osteotomy guide in the guide fiducial marker coordinate system and the position *T*₃ of the surface of the osteotomy guide in the guide fiducial marker coordinate system.

The corrected position and orientation of the osteotomy guide 4 relative to the guide fiducial marker 3 may be then used to replace the initial positional information recorded before delivery from the factory so as to instead serve as a basis for tracking the position of the osteotomy guide and controlling movement of the robotic arm during surgery. In this way, accurate positioning of the osteotomy guide can be achieved. Preferably, the control device further includes a storage device for storing information about the positional and orientational mapping of the guide fiducial marker 3 to the osteotomy guide 4. Further, the navigation device may be integrated with the control device to form a navigation system.

As shown in Fig. 7, the osteotomy guide block 40 may be provided therein with at least two correcting holes. In another embodiment, the checking hole 409 may provide one of the correcting holes. In the illustrated embodiment, apart from the checking hole 409, two correcting holes are separately provided, which are a first correcting hole 414a and a second correcting hole 414b. The present invention is not limited to any particular position of each correcting hole, and they may be provided on the same surface, or on different surfaces. In addition, two checking fiducial markers 16 (also serving as the two correcting fiducial markers) are secured to any two of the first correcting hole 414a, the second correcting hole 414b and the checking hole 409. Through causing the step surfaces of the two checking fiducial markers 16 to be matched with the upper surface of the osteotomy guide, the positions and orientations of the two holes to which the checking fiducial markers are secured can be derived and taken as basis for further calculating the position and orientation of each guide slot or hole relative to the guide fiducial markers. In this way, the position of the guide relative to the guide fiducial marker is corrected. In some embodiments, an end face of each correcting hole, the upper surface of the osteotomy guide block and the step surfaces of the correcting fiducial markers may be coplanar. This allows for easier calculation and thus enhanced correction efficiency.

In this embodiment, positional information of each correcting fiducial marker in the guide fiducial marker coordinate system may include the position and orientation of a front end point of the correcting fiducial marker in the guide fiducial marker coordinate system and the position and orientation of the step surface of the correcting fiducial marker in the guide fiducial marker coordinate system.

Further, in order to enable osteotomies on left or right leg, the osteotomy guide block 40 may be an axisymmetric structure. Alternatively, as shown in Fig. 10, two osteotomy guide blocks 41 and 42 mirrored to each other may be provided. In this case, the osteotomy guide block 41 may be configured for osteotomy guidance for the left leg and the osteotomy guide block 42 for the right leg. In order to facilitate attachment and detachment, each of the osteotomy guide blocks may be connected to the eccentric crank 403 by a quick-change interface. In the illustrated embodiment, the osteotomy guide block 41 is connected to the eccentric crank 403 by a quick-change interface 415, and the osteotomy guide block 42 is connected to the eccentric crank 403 by a quick-change interface 421. Optionally, the two quick-change interfaces 415 and 421 may be arranged in positional correspondence with, rather than mirrored to, each other.

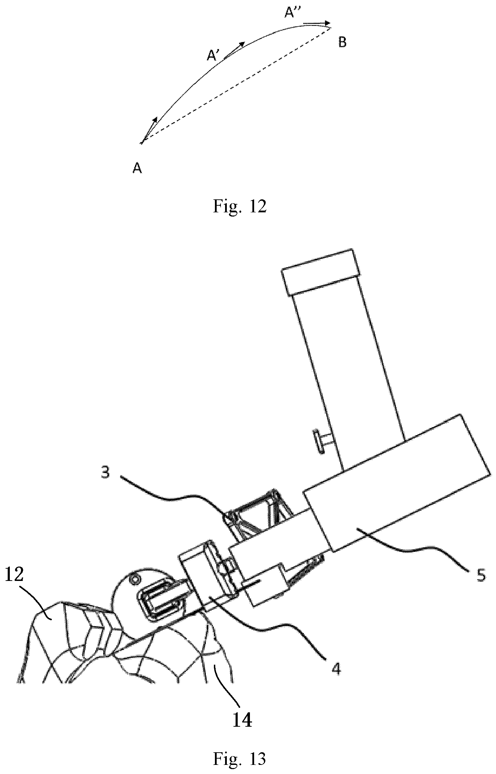

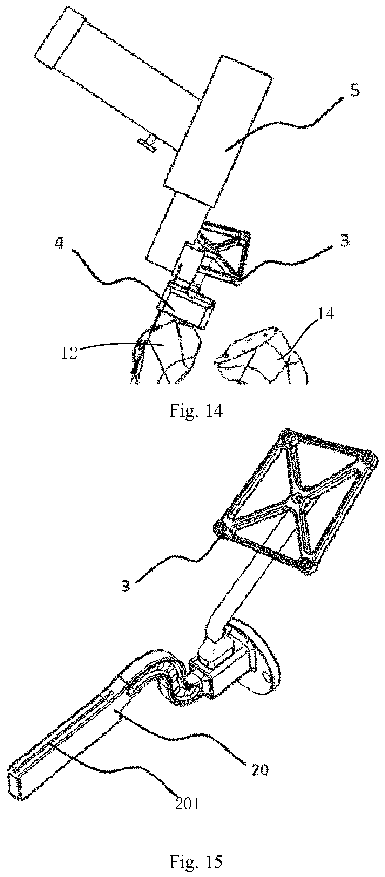

As shown in Fig. 11, in other embodiments, only one osteotomy guide block 43 is provided, but the osteotomy guide block 43 has two quick-change interfaces 431 and 432 disposed on its opposite sides of the osteotomy guide block 43. The eccentric crank 403 is selectively connected in a detachable manner to one of the quick-change interfaces 431 and 432. In this way, osteotomies on any knee joint are also possible by selectively connecting the eccentric crank 403 to the respective quick-change interface of the osteotomy guide block. However, the present invention is not limited to any particular relative positions of the two quick-change interfaces 431 and 432, and they may be so disposed on the opposite surfaces that their axes are co-linear. Still alternatively, the osteotomy guide block 43 may have only one quick-change interface, and the osteotomy guide block 43 may be flipped 180 degrees and connected to the eccentric crank 403. In this way, the osteotomy guide block 43 is also usable for left or right leg. To sum up, osteotomies on left or right leg are made possible either with one or two osteotomy guide blocks, and the present invention is not limited in this regard. Of course, it is preferred that a single osteotomy guide block can be used for left or right leg. With an osteotomy on the left leg as an example, the osteotomy guide 4 of the present invention can be used to provide guidance both for tibial plateau preparation as shown in Fig. 13 and for femoral cutting as shown in Fig. 14 without the need to change over to another osteotomy guide during surgery. This results in improved surgical convenience and enhanced efficiency.

In embodiments of the present invention, the controller may be configured to provide a desired travel path defined by a plurality of positional points and to control the robotic arm 2 to move, thus driving the osteotomy guide 4 and the guide fiducial marker 3 to move so that the guide fiducial marker 3 follows the desired travel path to reach a desired position. This can result in an additional increased in positioning accuracy. The controller may be further configured to derive the desired travel path for the guide fiducial marker from the current and desired positions of the guide fiducial marker 3. Further, the controller may be configured to determine the desired position for the guide fiducial marker from both the positional and orientational mapping of the guide fiducial marker to the osteotomy guide and a target position for the osteotomy guide.

More specifically, as shown in Fig. 12, before an osteotomy procedure is performed, the controller may first determine the current position A of the guide fiducial marker 3 from registration information of the robotic arm 2 (containing the positional and orientational mapping of the guide fiducial marker to the osteotomy guide) and then determine a desired position B for the guide fiducial marker 3 from the patient's bone registration information (e.g., a 3D digital knee joint model and information derived from CT/MR scan images), information about suitable prostheses and the like. After that, by utilizing inverse kinematics, the controller may plan a desired travel path for the guide fiducial marker 3 and issue instructions to different sections of the robotic arm, which may cause the sections to move and thus drive the guide fiducial marker 3 to move in a controlled manner. During movement, the tracker 6 may track the position (e.g., A' and A") of the guide fiducial marker 3 in real time and feed it to the controller. When receiving the detected position, the controller may calculate its deviation from a desired position and, based thereon, update the travel path for the guide fiducial marker 3. Under such control, the guide fiducial marker 3 ultimately reaches the desired position B, realizing accurate positioning of the osteotomy guide 4.

The guide features on the osteotomy guide block will be described in greater below by way of specific examples.

As shown in Fig. 15, in a first example, an osteotomy guide is provided, which includes the osteotomy guide block 20 that is used for guidance for distal femoral cutting and tibial plateau preparation. More specifically, the osteotomy guide block 20 has only one guide feature, which is a 0° guide slot 201 provided in an upper surface of the osteotomy guide block 20. The osteotomy guide may be rotated to switch between the distal femoral cutting and tibial plateau preparation modes. It should be appreciated that, as used herein, the term "0° guide slot" refers to a slot extending in parallelism with, i.e., at an angle of 0° with respect to, side surfaces of the osteotomy guide block, for example, from the upper to lower surface of the osteotomy guide block. Likewise, the term "45° guide slot" refers to a slot extending at an angle of 45° with respect to the side surfaces of the osteotomy guide block, for example, from the upper to lower surface of the osteotomy guide block.

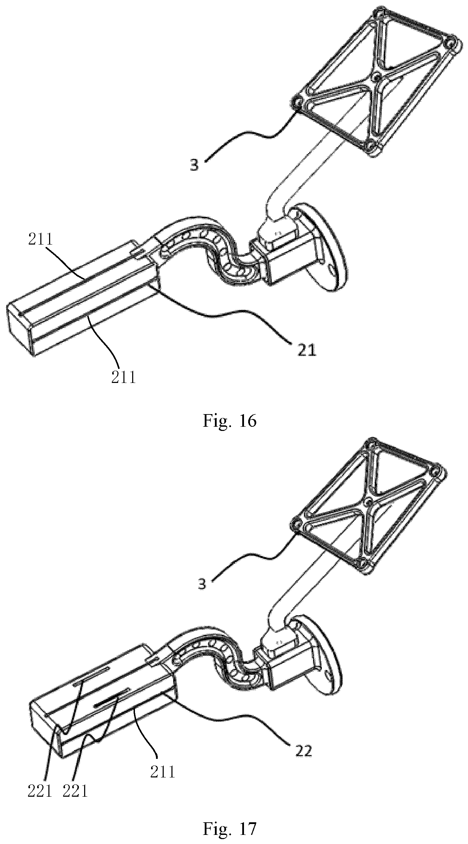

As shown in Fig. 16, in a second example, an osteotomy guide is provided, which includes the osteotomy guide block 21 that can be used for guidance for distal femoral cutting and tibial plateau preparation. The osteotomy guide block 21 has two guide features, which are both 0° guide slots 211 provided in different surfaces of the osteotomy guide block 21. For example, one of them may be provided in the upper surface and the other in a side surface adjacent to the upper surface. However, since the two 0° guide slots 211 are arranged around an axis of rotation of the osteotomy guide block 21, a surgical instrument 5 can switch from one bone surface to be cut to another by rotating a reduced angle. This avoids significant identification errors that may be caused by large movement strokes of the fiducial marker, as well as significant rotational errors that may be caused by excessive changes in the position and orientation of the robotic arm.

As shown in Fig. 17, in a third example, an osteotomy guide is provided, which includes the osteotomy guide block 22 that can be used for guidance for distal femoral cutting, tibial plateau preparation and trochlear cutting. Specifically, it adds two slots 221 respectively for cutting of the left and right trochlear grooves to the osteotomy guide block 21 of the second example.

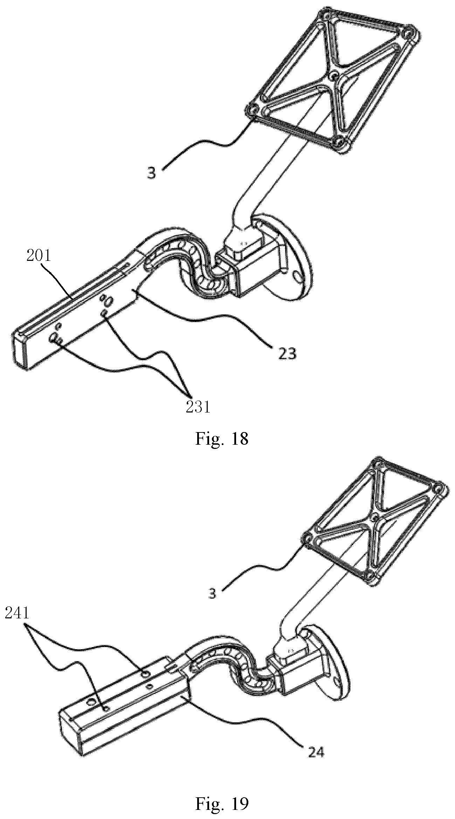

As shown in Fig. 18, in a fourth example, an osteotomy guide is provided, which includes the osteotomy guide block 23 that can be used for guidance for distal femoral cutting and tibial plateau preparation. It adds several guide holes 231 (e.g., two) that can be used for guidance for the drilling of mounting holes for femoral prostheses and tibial tools to the osteotomy guide block 20 of the first example.

As shown in Fig. 19, in a fifth example, an osteotomy guide is provided, which includes the osteotomy guide block 24 that can be used for guidance for distal femoral cutting and tibial plateau preparation. In addition to the two guide slots of the second example, it also has guide holes 241 (e.g., two) that can be used for guidance for the drilling of mounting holes for femoral prostheses and tibial tools.

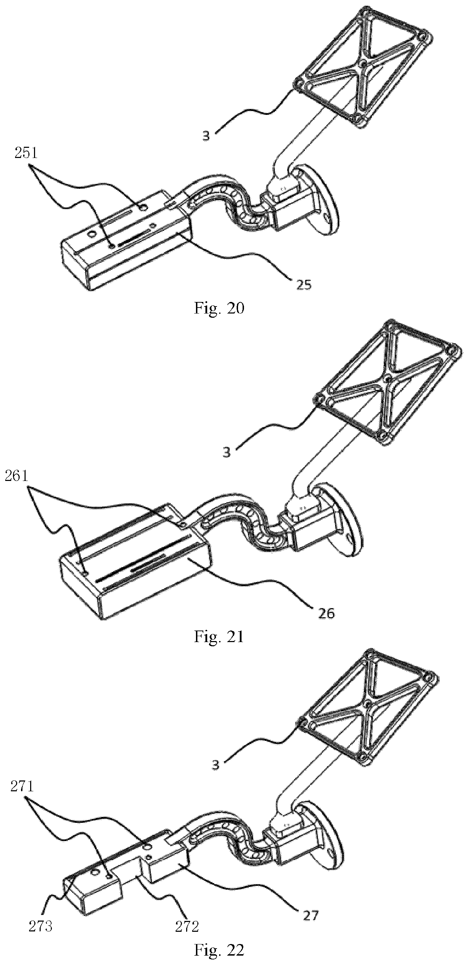

As shown in Fig. 20, in a sixth example, an osteotomy guide is provided, which includes the osteotomy guide block 25 that further has several guide holes 251 (e.g., two) in addition to the guide features of the third example. Therefore, the osteotomy guide block 25 can be used for guidance not only for distal femoral cutting, tibial plateau preparation and trochlear cutting but for drilling of mounting holes for femoral prostheses and tibial tools.

As shown in Fig. 21, in a seventh example, an osteotomy guide is provided, which includes the osteotomy guide block 26 that further has locating holes 261 in addition to other necessary guide features. When the osteotomy guide is placed at a predetermined location by the robotic arm 2, locating pins may be used to fix the osteotomy guide to the bone through the locating holes 261 of the osteotomy guide block 26, thus allowing anterior femoral, anterior femoral oblique, posterior femoral, posterior femoral oblique and trochlear cutting in a single procedure. This can avoid movement of the osteotomy guide during surgery due to insufficient stiffness of the robotic arm, thus reducing guide errors.

As shown in Fig. 22, in an eighth example, an osteotomy guide is provided, which includes the osteotomy guide block 27 that is a variant of that of the fifth example with two guide slots and two guide holes. Specifically, it has a rectangular notch 272 used for guidance for trochlear cutting, an elongate guide slot 273 used for guidance for the cutting of other femoral and tibial surfaces, and guide holes 271 used for guidance for drilling of mounting holes for femoral prostheses and tibial tools.

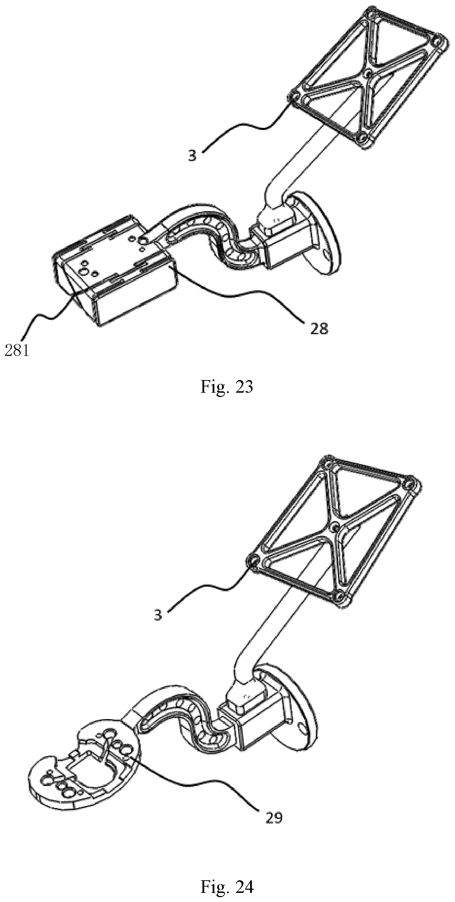

As shown in Fig. 23, in a ninth example, an osteotomy guide is provided, which includes the osteotomy guide block 28 that has a guide slot having openings 281 at one or both ends. The guide slot may be either a 0° or 45° guide slot. With the end opening(s) 281, a surgical instrument 5 such as a swing saw can travel within a larger range, allowing the guide block to have a reduced length. This can lower the requirements on applicable lesions and reduce damage to the patient's tissue. Here, the term "end opening" means that the guide slot extends at its one or both ends up to and through the respective side surface(s) of the osteotomy guide block and thus has one or more open ends.

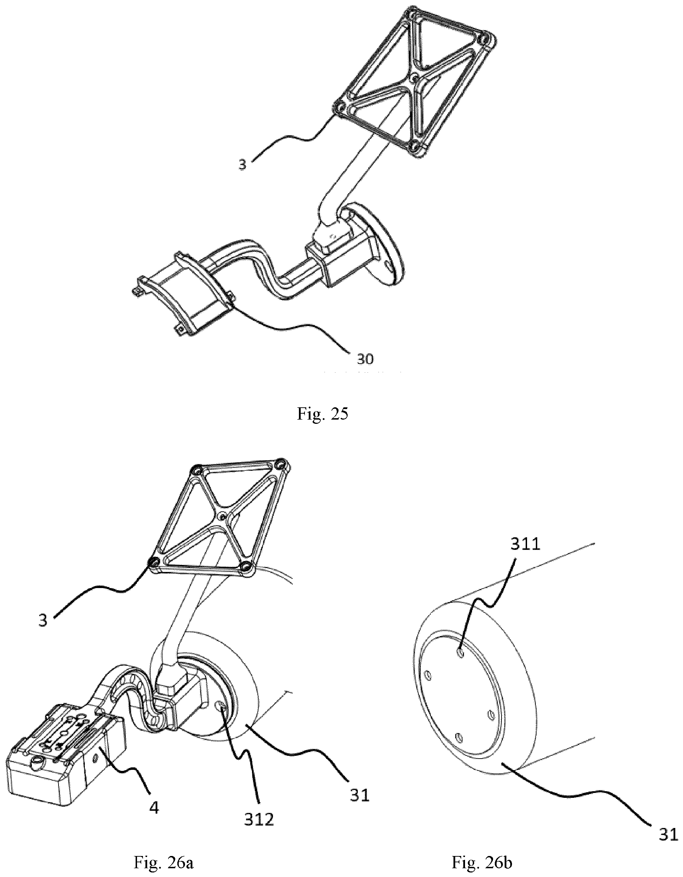

As shown in Fig. 24, in a tenth example, an osteotomy guide is provided, which includes the osteotomy guide block 29 that is a tibial tooling guide capable of automatic positioning of tibial tooling, differing from those of the above examples. As shown in Fig. 25, in an eleventh example, an osteotomy guide is provided, which includes the osteotomy guide block 30. What is different from the previous embodiment is that the trochlear cutting guide is used as the osteotomy guide block 30 to realize the automatic positioning of the trochlear cutting guide.

Thus, in various embodiments of the present invention, the osteotomy guide block may have different combinations of guide features as required by the practical applications. In some embodiments, both guide slots and holes may be provided on the osteotomy guide block, but the present invention is not limited to any particular number or positions of the guide slots on the osteotomy guide block. Likewise, the present invention is also not limited to any particular number or positions of the guide holes on the osteotomy guide block. The osteotomy guide block may be provided with any of 0° guide slots, 45° guide slots and trochlear cutting slots, or any combination thereof. Further, the osteotomy guide block may be provided with any of guide holes for the positioning of femoral prostheses, guide holes for the locating of left tibial tooling, guide holes for the locating of right tibial tooling and securing holes for the osteotomy guide, or any combination thereof.

Fig. 26a shows a sterile bag 31 for use on the robotic arm. For clarity, only part of the sterile bag 31 is shown. The sterile bag 31 may be disposed over the end section of the robotic arm so that its one end portion covers a connecting port (e.g., a flange) of the robotic arm. The connecting port may be detachably connected to the osteotomy guide. In this way, the surgical area can be isolated to ensure a sterile surgical environment. Preferably, an end portion of the sterile bag 31 where it is connected to the robotic arm's flange may be made of a material with a specified degree of hardness, such as PEEK plastic for medical use, and provided therein with passage holes 311 that allows passage of respective fasteners 312 for locking the osteotomy guide, such as screws and locating pins. The sterile bag 31 can support surgical instrument mounting and minimize the exposure to non-sterile portions of the robotic arm.

In embodiments not covered by the present invention and shown only for illustrative purpose, there is also provided a computer readable storage medium storing thereon instructions, which, when executed by a processor, cause the aforementioned controller to perform the steps in the method.

While the present invention has been described above with reference to preferred embodiments thereof, its scope is in no way limited to the above embodiments. For example, the present invention is not limited to any particular number or types of the guide features on the osteotomy guide block. When the orthopedic surgery system is used in other types of orthopedic surgery, other guide and holes than those described above may be included. Moreover, the present invention is neither limited to any particular position of the checking hole, nor limited to any particular positions of correcting holes. Further, in order to ensure accurate guidance and allow a reduced thickness of the osteotomy guide block, the guide holes are preferably provided in the same surface of the osteotomy guide block. Furthermore, the fiducial markers are preferred to be optical fiducial markers capable of emitting optical signals.

The foregoing description presents merely preferred embodiments of the present invention and is not intended to limit the scope of the present invention in any sense. It is intended that all changes and modifications made by those of ordinary skill in the art in light of the above teachings fall within the scope of the appended claims.

## Claims

1. An orthopedic surgery system, comprising a control device, a navigation device, a robotic arm (2) and an osteotomy guide (4), the osteotomy guide (4) attached to one end of the robotic arm (2), the robotic arm (2) configured to adjust the position and orientation of the osteotomy guide (4),
the navigation device comprising a tracker (6) and a guide fiducial marker (3), the guide fiducial marker (3) attached to the osteotomy guide (4) or the robotic arm (2), the tracker (6) configured to track the current position of the guide fiducial marker (3) and generate information about the current position, the position of the guide fiducial marker (3) configured to characterize the position of the osteotomy guide (4),
the control device configured to control the robotic arm (2) to move based both on the information about the current position of the guide fiducial marker (3) fed back from the tracker (6) and on information about a desired position for the guide fiducial marker (3), thereby driving the osteotomy guide (4) and the guide fiducial marker (3) to move so that the guide fiducial marker (3) reaches the desired position, **characterized in that**:
the orthopedic surgery system further comprises a checking device configured to check whether the osteotomy guide (4) and/or the guide fiducial marker (3) has/have undergone deformation, wherein:
the osteotomy guide (4) comprises an osteotomy guide block (40, 41, 42, 43, 20, 21, 22, 23, 24) provided thereon with a plurality of guide features for providing osteotomy guidance, and the checking device comprises at least one checking fiducial marker (16) configured for detachable attachment to the osteotomy guide block (40, 41, 42, 43, 20, 21, 22, 23, 24);
the tracker (6) is configured to record initial positional information of the checking fiducial marker (16) relative to the guide fiducial marker (3) before delivery from the factory and record information about the current position of the checking fiducial marker (16) relative to the guide fiducial marker (3) after each surgical use; and
the control device is configured to determine whether there is a match between the initial positional information of the checking fiducial marker (16) and the information about the current position of the checking fiducial marker (16), if there is a match, determine that the osteotomy guide (4) and/or the guide fiducial marker (3) has/have not undergone deformation, and if there is not a match, determine that the osteotomy guide (4) and/or the guide fiducial marker (3) has/have undergone deformation.

2. The orthopedic surgery system according to claim 1, further comprising a correcting device for correcting the position of the osteotomy guide (4) relative to the guide fiducial marker (3) when the checking device determines that the osteotomy guide (4) and/or the guide fiducial marker (3) has/have not undergone deformation, the correcting device comprising at least two correcting fiducial markers each configured for detachable attachment to the osteotomy guide block (40, 41, 42, 43, 20, 21, 22, 23, 24),
wherein the tracker (6) is configured to record positional information of the at least two correcting fiducial markers relative to the guide fiducial marker (3), and
wherein the control device is configured to derive the information about the current position of the osteotomy guide (4) relative to the guide fiducial marker (3) from the positional information of the at least two correcting fiducial markers relative to the guide fiducial marker (3) and update the position of the osteotomy guide (4) relative to the guide fiducial marker (3) using the derived information about the current position.

3. The orthopedic surgery system according to claim 2, wherein the positional information of the two correcting fiducial markers relative to the guide fiducial marker (3) is respectively denoted as *T*₁ and *T*₂, *T*₁ is represented by a positional representation of one of the two correcting fiducial markers in a coordinate system of the guide fiducial marker (3) and *T*₂ is represented by a positional representation of the other of the two correcting fiducial markers in the coordinate system of the guide fiducial marker (3),
the control device is configured to derive a positional representation *T*₀ of a center of an osteotomy guide block (40, 41, 42, 43, 20, 21, 22, 23, 24) in the coordinate system of the guide fiducial marker (3) and a positional representation *T*₃ of a surface of the osteotomy guide block (40, 41, 42, 43, 20, 21, 22, 23, 24) in the coordinate system of the guide fiducial marker (3) from the positional representations *T*₁ and *T*₂ of the at least two correcting fiducial markers in the coordinate system of the guide fiducial marker (3) and derive the position and orientation of the osteotomy guide (4) relative to the guide fiducial marker (3) from both the positional representation *T*₀ of the center of the osteotomy guide block (40, 41, 42, 43, 20, 21, 22, 23, 24) in the coordinate system of the guide fiducial marker (3) and the positional representation *T*₃ of the surface of the osteotomy guide block (40, 41, 42, 43, 20, 21, 22, 23, 24) in the coordinate system of the guide fiducial marker (3), and/or
wherein the positional information of the checking fiducial marker (16) relative to the guide fiducial marker (3) comprises the position and orientation of a front end point of the checking fiducial marker (16) in a coordinate system of the guide fiducial marker (3), and/or
wherein the position of each correcting fiducial marker in the coordinate system of the guide fiducial marker (3) comprises:
the position and orientation of a front end point of the correcting fiducial marker in the coordinate system of the guide fiducial marker (3); and
the position and orientation of a step surface of the correcting fiducial marker in the coordinate system of the guide fiducial marker (3), and
wherein the step surface is of a step of the correcting fiducial marker and is parallel to a surface of the osteotomy guide block (40, 41, 42, 43, 20, 21, 22, 23, 24).

4. The orthopedic surgery system according to claim 1, wherein the navigation device further comprises a base fiducial marker (15) which is maintained stationary, and the position of the guide fiducial marker (3) is relative to that of the base fiducial marker (15), and/or
wherein the control device is configured to provide a desired travel path defined by a plurality of positional points and to control the robotic arm (2) to move so that the guide fiducial marker (3) follows the desired travel path to reach the desired position.

5. The orthopedic surgery system according to claim 1, further comprising a storage device for storing a positional and orientational mapping between the guide fiducial marker (3) and the osteotomy guide (4).

6. The orthopedic surgery system according to claim 5, wherein the osteotomy guide (4) comprises an osteotomy guide block (40, 41, 42, 43, 20, 21, 22, 23, 24) provided thereon with a number of guide features for providing multiple osteotomy guidance modes, and wherein the positional and orientational mapping between the guide fiducial marker (3) and the osteotomy guide (4) comprises positional and orientational mappings between the guide features and the guide fiducial marker (3).

7. The orthopedic surgery system according to claim 6, wherein the positional and orientational mapping between each of the guide features and the guide fiducial marker (3) is determined by:
determining a relative positional relationship among fiducial marker (3) balls on the guide fiducial marker (3) and establishing a coordinate system of the guide fiducial marker (3) based on the relative positional relationship;
determining positional information of a center of an osteotomy guide block (40, 41, 42, 43, 20, 21, 22, 23, 24) in the coordinate system of the guide fiducial marker (3); and
determining the position and orientation of the guide feature in the coordinate system of the guide fiducial marker (3) from both positional information of the guide feature relative to the center of the osteotomy guide block (40, 41, 42, 43, 20, 21, 22, 23, 24) and the positional information of the center of the osteotomy guide block (40, 41, 42, 43, 20, 21, 22, 23, 24) in the coordinate system of the guide fiducial marker (3).

8. The orthopedic surgery system according to claim 1, wherein the osteotomy guide block (40, 41, 42, 43, 20, 21, 22, 23, 24) is provided therein with at least one checking hole (409) to which the respective checking fiducial marker(s) is/are secured, and wherein each checking fiducial marker (16) has a step with a step surface that is parallel to a surface of the osteotomy guide block (40, 41, 42, 43, 20, 21, 22, 23, 24).

9. The orthopedic surgery system according to claim 8, wherein the osteotomy guide block (40, 41, 42, 43, 20, 21, 22, 23, 24) has an upper surface in which the guide features are provided, wherein the checking hole (409) is vertically provided in the upper surface, and wherein the step surface is matched with the upper surface, and/or
wherein the osteotomy guide block (40, 41, 42, 43, 20, 21, 22, 23, 24) is provided therein with a correcting hole (414a, 414b), and wherein two correcting fiducial markers are respectively secured to the correcting hole (414a, 414b), or two correcting fiducial markers are respectively secured to one checking hole (409) and the correcting hole (414a, 414b).

10. The orthopedic surgery system according to claim 9, wherein an axis line of the checking hole (409) is located in a plane of symmetry of the osteotomy guide block (40, 41, 42, 43, 20, 21, 22, 23, 24), and wherein an end face of the checking hole (409), the upper surface of the osteotomy guide block (40, 41, 42, 43, 20, 21, 22, 23, 24) and the step surface are co-planar.

11. The orthopedic surgery system according to claim 8, wherein the osteotomy guide block (40, 41, 42, 43, 20, 21, 22, 23, 24) is provided therein with a correcting hole (414a, 414b), and wherein two correcting fiducial markers are respectively secured to the correcting hole (414a, 414b), or two correcting fiducial markers are respectively secured to one checking hole (409) and the correcting hole (414a, 414b),
wherein each correcting hole (414a, 414b) has an end face that is co-planar with both the surface of the osteotomy guide block (40, 41, 42, 43, 20, 21, 22, 23, 24) and a step surface of each correcting fiducial marker.

12. The orthopedic surgery system according to claim 1, wherein the osteotomy guide (4) comprises an osteotomy guide block (40, 41, 42, 43, 20, 21, 22, 23, 24) and a mounting interface (401), the mounting interface (401) connected to the osteotomy guide block (40, 41, 42, 43, 20, 21, 22, 23, 24) by a connecting shaft, the osteotomy guide block (40, 41, 42, 43, 20, 21, 22, 23, 24) provided thereon with a plurality of guide features for providing osteotomy guidance, the mounting interface (401) attached detachably to the end of the robotic arm (2).

13. The orthopedic surgery system according to claim 12, wherein the connecting shaft is an eccentric crank (403) that is connected to both the osteotomy guide block (40, 41, 42, 43, 20, 21, 22, 23, 24) and the mounting interface (401) so that an axis of rotation of the mounting interface (401) is offset from an axis of rotation of the osteotomy guide block (40, 41, 42, 43, 20, 21, 22, 23, 24).

14. The orthopedic surgery system according to claim 13, wherein the osteotomy guide (4) further comprises a holder (402), wherein the osteotomy guide block (40, 41, 42, 43, 20, 21, 22, 23, 24), the eccentric crank (403), the holder (402) and the mounting interface (401) are sequentially connected together in sequence, and wherein the holder (402) is provided therein with fiducial marker mounting holes (4021), and/or
wherein the osteotomy guide block (40, 41, 42, 43, 20, 21, 22, 23, 24) is attached detachably to the eccentric crank (403).

15. The orthopedic surgery system according to claim 12, wherein the guide features include a number of guide slots provided on a single surface of the osteotomy guide block (40, 41, 42, 43, 20, 21, 22, 23, 24), or on different surface around an axis line of the osteotomy guide block (40, 41, 42, 43, 20, 21, 22, 23, 24).

16. The orthopedic surgery system according to claim 13, wherein the osteotomy guide (4) comprises two osteotomy guide blocks (40, 41, 42, 43, 20, 21, 22, 23, 24) mirrored to each other and each provided thereon with a mounting interface (401) configured for detachable connection with the eccentric crank (403), or
wherein the osteotomy guide (4) comprises one osteotomy guide block (40, 41, 42, 43, 20, 21, 22, 23, 24) provided thereon with two mounting interfaces (401) arranged in opposition to each other, and wherein the eccentric crank (403) is selectively connected detachably to one of the mounting interfaces (401).

17. The orthopedic surgery system according to claim 1, further comprising a sterile bag (31) disposed over the end of the robotic arm (2) so that an end portion of the sterile bag (31) covers a connecting port of the robotic arm (2), the connecting port connected detachably to the osteotomy guide (4).

18. The orthopedic surgery system according to claim 17, wherein the sterile bag (31) is provided in one end thereof with passage holes (311).

19. An osteotomy guide (4) for use in the orthopedic surgery system according to claim 1, the osteotomy guide (4) comprising an osteotomy guide block (40, 41, 42, 43, 20, 21, 22, 23, 24), a connecting shaft and a mounting interface (401), the osteotomy guide block (40, 41, 42, 43, 20, 21, 22, 23, 24) provided thereon with a number of guide features for providing multiple osteotomy guidance modes, both ends of the connecting shaft connected to both the osteotomy guide block (40, 41, 42, 43, 20, 21, 22, 23, 24) and the mounting interface (401), the mounting interface (401) configured for detachable connection with one end of a robotic arm (2).

20. The osteotomy guide (4) according to claim 19, wherein the guide features include guide slot(s) and guide hole(s) (231, 241, 251, 271), and/or
wherein the connecting shaft is an eccentric crank (403).

21. The osteotomy guide (4) according to claim 20, wherein each guide slot extends through a surface of the osteotomy guide block (40, 41, 42, 43, 20, 21, 22, 23, 24) and thus has end openings (281), and/or wherein each guide slot is flared in shape, and/or
wherein a plurality of guide slots are included and arranged in different surfaces around an axis line of the osteotomy guide block (40, 41, 42, 43, 20, 21, 22, 23, 24).

22. The osteotomy guide (4) according to claim 20, wherein the guide slot(s) include(s) at least one of 0° guide slot(s) (407, 411), 45° guide slot(s) (408, 410) and trochlear cutting guide slot(s) (221), and wherein the guide hole(s) include(s) at least one of guide hole(s) for the mounting of a femoral prosthesis (404), guide hole(s) for the locating of left tibial tooling (406), guide hole(s) for the locating of right tibial tooling (413) and securing hole(s) for the osteotomy guide (4), or
wherein the guide slot(s) include(s) two 0° guide slots (201, 211), two 45° guide slots and two trochlear cutting guide slots (221).

23. The osteotomy guide (4) according to claim 19, wherein the osteotomy guide block (40, 41, 42, 43, 20, 21, 22, 23, 24) is an axisymmetric structure, and/or
wherein the osteotomy guide block (40, 41, 42, 43, 20, 21, 22, 23, 24) is provided therein with a checking hole (409) and/or a correcting hole (414a, 414b).

## Patentansprüche

1. Orthopädisches Chirurgiesystem, umfassend eine Steuerungsvorrichtung, eine Navigationsvorrichtung, einen Roboterarm (2) und eine Osteotomieführung (4), wobei die Osteotomieführung (4) an einem Ende des Roboterarms (2) befestigt ist, wobei der Roboterarm (2), ausgebildet ist, die Position und Ausrichtung der Osteotomieführung (4) einzustellen,
wobei die Navigationsvorrichtung einen Tracker (6) und einen Führungsreferenzmarker (3) umfasst, wobei der Führungsreferenzmarker (3) an der Osteotomieführung (4) oder dem Roboterarm (2) befestigt ist, wobei der Tracker (6) ausgebildet ist, eine aktuelle Position des Führungsreferenzmarkers (3) zu tracken und Informationen über die aktuelle Position zu generieren, wobei die Position des Führungsreferenzmarkers (3) ausgebildet ist, die Position der Osteotomieführung (4) zu charakterisieren,
wobei die Steuervorrichtung ausgebildet ist, den Roboterarm (2) zur Bewegung sowohl basierend auf den Informationen über die aktuelle Position des Führungsreferenzmarkers (3), die von dem Tracker (6) zurückgemeldet werden, als auch auf Informationen über eine gewünschte Position für den Führungsreferenzmarker (3) zu steuern, wodurch die Osteotomieführung (4) und der Führungsreferenzmarker (3) zur Bewegung angesteuert werden, so dass der Führungsreferenzmarker (3) die gewünschte Position erreicht, **dadurch gekennzeichnet, dass**:
das orthopädische Chirurgiesystem ferner eine Kontrollvorrichtung umfasst, welche ausgebildet ist, zu kontrollieren, ob die Osteotomieführung (4) und/oder der Führungsreferenzmarker (3) eine Verformung erfahren hat/haben, wobei:
die Osteotomieführung (4) einen Osteotomieführungsblock (40, 41, 42, 43, 20, 21, 22, 23, 24) umfasst, der darauf mit einer Vielzahl von Führungsmerkmalen zur Bereitstellung einer Osteotomieführung versehen ist, und wobei die Kontrollvorrichtung mindestens einen Kontrollreferenzmarker (16) umfasst, der zur lösbaren Befestigung an dem Osteotomieführungsblock (40, 41, 42, 43, 20, 21, 22, 23, 24) ausgebildet ist;
wobei der Tracker (6) ausgebildet ist, anfängliche Positionsinformationen des Kontrollreferenzmarkers (16) relativ zu dem Führungsreferenzmarker (3) vor der Auslieferung aus der Fabrik aufzuzeichnen und Informationen über die aktuelle Position des Kontrollreferenzmarkers (16) relativ zu dem Führungsreferenzmarker (3) nach jedem chirurgischen Einsatz aufzuzeichnen; und
wobei die Steuervorrichtung ausgebildet ist, zu bestimmen, ob eine Übereinstimmung zwischen den anfänglichen Positionsinformationen des Kontrollreferenzmarkers (16) und den Informationen über die aktuelle Position des Kontrollreferenzmarkers (16) besteht, zu bestimmen, falls eine Übereinstimmung vorliegt, dass die Osteotomieführung (4) und/oder der Führungsreferenzmarker (3) keine Verformung erfahren hat/haben, und zu bestimmen, falls keine Übereinstimmung vorliegt, dass die Osteotomieführung (4) und/oder die Führungsreferenzmarker (3) eine Verformung erfahren hat/haben.

2. Orthopädisches Chirurgiesystem nach Anspruch 1, ferner umfassend eine Korrekturvorrichtung zum Korrigieren der Position der Osteotomieführung (4) relativ zu dem Führungsreferenzmarker (3), wenn die Kontrollvorrichtung feststellt, dass die Osteotomieführung (4) und/oder oder des Führungsreferenzmarkers (3) keine Verformung erfahren hat/haben, wobei die Korrekturvorrichtung mindestens zwei Korrekturreferenzmarker umfasst, die jeweils zur lösbaren Befestigung an dem Osteotomieführungsblock (40, 41, 42, 43, 20, 21, 22, 23, 24) ausgebildet sind,
wobei der Tracker (6) ausgebildet ist, Positionsinformationen der mindestens zwei Korrekturreferenzmarker relativ zu dem Führungsreferenzmarker (3) aufzuzeichnen, und
wobei die Steuerungsvorrichtung ausgebildet ist, die Informationen über die aktuelle Position der Osteotomieführung (4) relativ zu dem Führungsreferenzmarker (3) aus den Positionsinformationen der mindestens zwei Korrekturreferenzmarker relativ zu dem Führungsreferenzmarker (3) abzuleiten und die Position der Osteotomieführung (4) relativ zu dem Führungsreferenzmarker (3) unter Verwendung der abgeleiteten Informationen über die aktuelle Position zu aktualisieren.

3. Orthopädisches Chirurgiesystem nach Anspruch 2, wobei die Positionsinformationen der zwei Korrekturreferenzmarker relativ zu dem Führungsreferenzmarker (3) als *T*₁ bzw. *T*₂ bezeichnet werden, wobei *T*₁ durch eine Positionsdarstellung einer der zwei Korrekturreferenzmarker in einem Koordinatensystem des Führungsreferenzmarkers (3) dargestellt wird, und wobei *T*₂ durch eine Positionsdarstellung des anderen der zwei Korrekturreferenzmarker in dem Koordinatensystem des Führungsreferenzmarkers (3) dargestellt wird,
wobei die Steuervorrichtung ausgebildet ist, eine Positionsdarstellung *T*₀ eines Zentrums eines Osteotomieführungsblocks (40, 41, 42, 43, 20, 21, 22, 23, 24) in dem Koordinatensystem des Führungsreferenzmarkers (3) und eine Positionsdarstellung *T*₃ einer Fläche des Osteotomieführungsblocks (40, 41, 42, 43, 20, 21, 22, 23, 24) in dem Koordinatensystem des Führungsreferenzmarkers (3) aus den Positionsdarstellungen *T*₁ und *T*₂ der mindestens zwei Korrekturreferenzmarker in dem Koordinatensystem des Führungsreferenzmarkers (3) abzuleiten, und die Position und Ausrichtung der Osteotomieführung (4) relativ zu dem Führungsreferenzmarker (3) aus der Positionsdarstellung *T*₀ des Zentrums des Osteotomieführungsblocks (40, 41, 42, 43, 20, 21, 22, 23, 24) in dem Koordinatensystem des Führungsreferenzmarkers (3) und die Positionsdarstellung *T*₃ der Fläche des Osteotomieführungsblocks (40, 41, 42, 43, 20, 21, 22, 23, 24) in dem Koordinatensystem des Führungsreferenzmarkers (3) abzuleiten, und/oder
wobei die Positionsinformationen des Kontrollreferenzmarkers (16) relativ zu dem Führungsreferenzmarker (3) die Position und Ausrichtung eines vorderen Endpunkts des Kontrollreferenzmarkers (16) in einem Koordinatensystem des Führungsreferenzmarkers (3) umfassen, und/oder
wobei die Position jedes Korrekturreferenzmarkers in dem Koordinatensystem des Führungsreferenzmarkers (3) umfasst:
die Position und Ausrichtung eines vorderen Endpunkts des Korrekturreferenzmarkers in dem Koordinatensystem des Führungsreferenzmarkers (3); und
die Position und Ausrichtung einer Stufenfläche des Korrekturreferenzmarkers in dem Koordinatensystem des Führungsreferenzmarkers (3), und
wobei die Stufenfläche eine Stufe des Korrekturreferenzmarkers ist und parallel zu einer Fläche des Osteotomieführungsblocks (40, 41, 42, 43, 20, 21, 22, 23, 24) ist.

4. Orthopädisches Chirurgiesystem nach Anspruch 1, wobei die Navigationsvorrichtung ferner einen Basisreferenzmarker (15) umfasst, der stationär gehalten wird, und wobei die Position des Führungsreferenzmarkers (3) relativ zu der des Basisreferenzmarkers (15) ist, und/oder
wobei die Steuervorrichtung ausgebildet ist, einen gewünschten Bewegungspfad bereitzustellen, der durch eine Vielzahl von Positionspunkten definiert ist, und den Roboterarm (2) zur Bewegung zu steuern, so dass der Führungsreferenzmarker (3) dem gewünschten Bewegungspfad folgt, um die gewünschte Position zu erreichen.

5. Orthopädisches Chirurgiesystem nach Anspruch 1, ferner umfassend eine Speichervorrichtung zum Speichern einer Positions- und Orientierungsabbildung zwischen dem Führungsreferenzmarker (3) und der Osteotomieführung (4).

6. Orthopädisches Chirurgiesystem nach Anspruch 5, wobei die Osteotomieführung (4) einen Osteotomieführungsblock (40, 41, 42, 43, 20, 21, 22, 23, 24) umfasst, der darauf mit einer Reihe von Führungsmerkmalen zur Bereitstellung mehrerer Osteotomieführungsmodi versehen ist, und wobei die Positions- und Orientierungsabbildung zwischen dem Führungsreferenzmarker (3) und der Osteotomieführung (4) Positions- und Orientierungsabbildungen zwischen den Führungsmerkmalen und dem Führungsreferenzmarker (3) umfasst.

7. Orthopädisches Chirurgiesystem nach Anspruch 6, wobei die Positions- und Orientierungsabbildung zwischen jedem der Führungsmerkmale und dem Führungsreferenzmarker (3) bestimmt wird durch:
Bestimmen einer relativen Positionsbeziehung zwischen Kugeln des Führungsreferenzmarkers (3) auf dem Führungsreferenzmarker (3) und Erstellen eines Koordinatensystems des Führungsreferenzmarkers (3) basierend auf der relativen Positionsbeziehung;
Bestimmen von Positionsinformationen eines Zentrums eines Osteotomieführungsblocks (40, 41, 42, 43, 20, 21, 22, 23, 24) in dem Koordinatensystem des Führungsreferenzmarkers (3); und
Bestimmen der Position und Ausrichtung des Führungsmerkmals in dem Koordinatensystem des Führungsreferenzmarkers (3) aus sowohl den Positionsinformationen des Führungsmerkmals relativ zu dem Zentrum des Osteotomieführungsblocks (40, 41, 42, 43, 20, 21, 22, 23, 24) als auch den Positionsinformationen des Zentrums des Osteotomieführungsblocks (40, 41, 42, 43, 20, 21, 22, 23, 24) in dem Koordinatensystem des Führungsreferenzmarkers (3).

8. Orthopädisches Chirurgiesystem nach Anspruch 1, wobei der Osteotomieführungsblock (40, 41, 42, 43, 20, 21, 22, 23, 24) darin mit mindestens einem Kontrollloch (409) versehen ist, mit welchem der/die jeweilige(n) Kontrollreferenzmarker befestigt ist/sind, und wobei jeder Kontrollreferenzmarker (16) eine Stufe mit einer Stufenfläche aufweist, die parallel zu einer Fläche des Osteotomieführungsblocks (40, 41, 42, 43, 20, 21, 22, 23, 24) ist.

9. Orthopädisches Chirurgiesystem nach Anspruch 8, wobei der Osteotomieführungsblock (40, 41, 42, 43, 20, 21, 22, 23, 24) eine obere Fläche aufweist, in der die Führungsmerkmale versehen sind, wobei das Kontrollloch (409) vertikal in der oberen Fläche versehen ist, und wobei die Stufenfläche mit der oberen Fläche übereinstimmt, und/oder
wobei der Osteotomieführungsblock (40, 41, 42, 43, 20, 21, 22, 23, 24) darin mit einem Korrekturloch (414a, 414b) versehen ist, und wobei zwei Korrekturreferenzmarker jeweils an dem Korrekturloch (414a, 414b) befestigt sind, oder wobei zwei Korrekturreferenzmarker jeweils an einem Kontrollloch (409) und dem Korrekturloch (414a, 414b) befestigt sind.

10. Orthopädisches Chirurgiesystem nach Anspruch 9, wobei eine Achsenlinie des Kontrolllochs (409) in einer Symmetrieebene des Osteotomieführungsblocks (40, 41, 42, 43, 20, 21, 22, 23, 24) liegt, und wobei eine Endfläche des Kontrolllochs (409), die obere Fläche des Osteotomieführungsblocks (40, 41, 42, 43, 20, 21, 22, 23, 24) und die Stufenfläche koplanar sind.

11. Orthopädisches Chirurgiesystem nach Anspruch 8, wobei der Osteotomieführungsblock (40, 41, 42, 43, 20, 21, 22, 23, 24) darin mit einem Korrekturloch (414a, 414b) versehen ist, und wobei an dem Korrekturloch (414a, 414b) jeweils zwei Korrekturreferenzmarker befestigt sind, oder wobei jeweils an einem Kontrollloch (409) und dem Korrekturloch (414a, 414b) zwei Korrekturreferenzmarker befestigt sind,
wobei jedes Korrekturloch (414a, 414b) eine Endfläche aufweist, die koplanar sowohl mit der Fläche des Osteotomieführungsblocks (40, 41, 42, 43, 20, 21, 22, 23, 24) als auch mit einer Stufenfläche jedes Korrekturreferenzmarkers ist.

12. Orthopädisches Chirurgiesystem nach Anspruch 1, wobei die Osteotomieführung (4) einen Osteotomieführungsblock (40, 41, 42, 43, 20, 21, 22, 23, 24) und eine Befestigungsschnittstelle (401) umfasst, wobei die Befestigungsschnittstelle (401) über eine Verbindungswelle mit dem Osteotomieführungsblock (40, 41, 42, 43, 20, 21, 22, 23, 24) verbunden ist, wobei der Osteotomieführungsblock (40, 41, 42, 43, 20, 21, 22, 23, 24) darauf mit einer Vielzahl von Führungsmerkmalen zur Bereitstellung einer Osteotomieführung versehen ist, wobei die Befestigungsschnittstelle (401) abnehmbar an dem Ende des Roboterarms (2) befestigt ist.

13. Orthopädisches Chirurgiesystem nach Anspruch 12, wobei die Verbindungswelle eine Exzenterkurbel (403) ist, die sowohl mit dem Osteotomieführungsblock (40, 41, 42, 43, 20, 21, 22, 23, 24) als auch mit der Befestigungsschnittstelle (401) verbunden ist, so dass eine Rotationsachse der Befestigungsschnittstelle (401) zu einer Rotationsachse des Osteotomieführungsblocks (40, 41, 42, 43, 20, 21, 22, 23, 24) versetzt ist.

14. Orthopädisches Chirurgiesystem nach Anspruch 13, wobei die Osteotomieführung (4) ferner einen Halter (402) umfasst, wobei der Osteotomieführungsblock (40, 41, 42, 43, 20, 21, 22, 23, 24), die Exzenterkurbel (403), der Halter (402) und die Befestigungsschnittstelle (401) sequenziell in Sequenz miteinander verbunden sind, und wobei der Halter (402) darin mit Referenzmarkerbefestigungslöchern (4021) versehen ist, und/oder
wobei der Osteotomieführungsblock (40, 41, 42, 43, 20, 21, 22, 23, 24) abnehmbar an der Exzenterkurbel (403) befestigt ist.

15. Orthopädisches Chirurgiesystem nach Anspruch 12, wobei die Führungsmerkmale eine Anzahl von Führungsschlitzen umfassen, die auf einer einzelnen Fläche des Osteotomieführungsblocks (40, 41, 42, 43, 20, 21, 22, 23, 24) oder auf einer anderen Fläche um eine Achsenlinie des Osteotomieführungsblocks (40, 41, 42, 43, 20, 21, 22, 23, 24) versehen sind.

16. Orthopädisches Chirurgiesystem nach Anspruch 13, wobei die Osteotomieführung (4) zwei Osteotomieführungsblöcke (40, 41, 42, 43, 20, 21, 22, 23, 24) umfasst, die zueinander gespiegelt sind und jeweils daran mit einer Befestigungsschnittstelle (401) versehen sind, die zur lösbaren Verbindung mit der Exzenterkurbel (403) ausgebildet ist, oder
wobei die Osteotomieführung (4) einen Osteotomieführungsblock (40, 41, 42, 43, 20, 21, 22, 23, 24) umfasst, der darauf mit zwei einander gegenüberliegend angeordneten Befestigungsschnittstellen (401) versehen ist, und wobei die Exzenterkurbel (403) selektiv lösbar mit einer der Befestigungsschnittstellen (401) verbunden ist.

17. Orthopädisches Chirurgiesystem nach Anspruch 1, ferner umfassend einen sterilen Beutel (31), der über dem Ende des Roboterarms (2) angeordnet ist, so dass ein Endabschnitt des sterilen Beutels (31) einen Verbindungsanschluss des Roboterarms (2) bedeckt, wobei der Verbindungsanschluss lösbar mit der Osteotomieführung (4) verbunden ist.

18. Orthopädisches Chirurgiesystem nach Anspruch 17, wobei der sterile Beutel (31) an einem Ende davon mit Durchgangslöchern (311) versehen ist.

19. Osteotomieführung (4) zur Verwendung in dem orthopädischen Chirurgiesystem nach Anspruch 1, wobei die Osteotomieführung (4) einen Osteotomieführungsblock (40, 41, 42, 43, 20, 21, 22, 23, 24), einen Verbindungsschaft und einer Befestigungsschnittstelle (401) umfasst, wobei der Osteotomieführungsblock (40, 41, 42, 43, 20, 21, 22, 23, 24) darauf mit einer Anzahl von Führungsmerkmalen versehen ist, um mehrere Osteotomieführungsmodi bereitzustellen, wobei beide Enden des Verbindungsschafts sowohl mit dem Osteotomieführungsblock (40, 41, 42, 43, 20, 21, 22, 23, 24) als auch mit der Befestigungsschnittstelle (401) verbunden sind, wobei die Befestigungsschnittstelle (401) für eine lösbare Verbindung mit einem Ende eines Roboterarms (2) ausgebildet ist.

20. Osteotomieführung (4) nach Anspruch 19, wobei die Führungsmerkmale einen Führungsschlitz / Führungsschlitze und ein Führungsloch / Führungslöcher (231, 241, 251, 271) umfassen, und/oder
wobei die Verbindungswelle eine Exzenterkurbel (403) ist.

21. Osteotomieführung (4) nach Anspruch 20, wobei sich jeder Führungsschlitz durch eine Fläche des Osteotomieführungsblocks (40, 41, 42, 43, 20, 21, 22, 23, 24) erstreckt und somit Endöffnungen aufweist (281), und/oder wobei jeder Führungsschlitz eine aufgeweitete Form aufweist, und/oder
wobei eine Vielzahl von Führungsschlitzen umfasst und in verschiedenen Flächen um eine Achsenlinie des Osteotomieführungsblocks (40, 41, 42, 43, 20, 21, 22, 23, 24) angeordnet sind.

22. Osteotomieführung (4) nach Anspruch 20, wobei der/die Führungsschlitz(e) mindestens einen von einem 0°-Führungsschlitz / 0°-Führungsschlitzen (407, 411), einem 45°-Führungsschlitz / 45°-Führungsschlitzen (408, 410) und einem Trochleaschnitt-Führungsschlitz / Trochleaschnitt-Führungsschlitzen (221) umfasst/umfassen, und wobei das Führungsloch / die Führungslöcher mindestens eines von einem Führungsloch / Führungslöcher für die Befestigung einer Femurprothese (404), einem Führungsloch / Führungslöcher für die Positionierung eines linken Tibia-Werkzeugs (406), einem Führungsloch / Führungslöcher für die Positionierung eines rechten Tibia-Werkzeugs (413) und einem Befestigungsloch / Befestigungslöcher für die Osteotomieführung (4) umfasst/umfassen, oder
wobei der/die Führungsschlitz(e) zwei 0°-Führungsschlitze (201, 211), zwei 45°-Führungsschlitze und zwei Trochleaschnitt-Führungsschlitze (221) umfasst/umfassen.

23. Osteotomieführung (4) nach Anspruch 19, wobei der Osteotomieführungsblock (40, 41, 42, 43, 20, 21, 22, 23, 24) eine achsensymmetrische Struktur ist und/oder
wobei der Osteotomieführungsblock (40, 41, 42, 43, 20, 21, 22, 23, 24) darin mit einem Kontrollloch (409) und/oder einem Korrekturloch (414a, 414b) versehen ist.

## Revendications

1. Système de chirurgie orthopédique, comprenant un dispositif de commande, un dispositif de navigation, un bras robotique (2) et un guide d'ostéotomie (4), le guide d'ostéotomie (4) fixé à une extrémité du bras robotique (2), le robot bras (2) configuré pour ajuster la position et l'orientation du guide d'ostéotomie (4),
le dispositif de navigation comprenant un tracker (6) et un marqueur de repère de guidage (3), le marqueur de repère de guide (3) fixé au guide d'ostéotomie (4) ou au bras robotique (2), le tracker (6) configuré pour suivre le position actuelle du marqueur de repère de guidage (3) et générer des informations sur la position actuelle, la position du marqueur de repère de guide (3) configurée pour caractériser la position du guide d'ostéotomie (4),
le dispositif de commande est configuré pour commander le mouvement du bras robotique (2) sur la base à la fois des informations concernant la position actuelle du marqueur de repère de guidage (3) renvoyées par le dispositif de suivi (6) et des informations concernant une position souhaitée pour le repère de guidage marqueur (3), entraînant ainsi le déplacement du guide d'ostéotomie (4) et du marqueur de repère de guidage (3) de sorte que le marqueur de repère de guidage (3) atteigne la position souhaitée, **caractérisé en ce que** :
le système de chirurgie orthopédique comprend en outre un dispositif de contrôle configuré pour vérifier si le guide d'ostéotomie (4) et/ou le marqueur de repère de guide (3) a/ont subi une déformation, dans lequel :
le guide d'ostéotomie (4) comprend un bloc de guidage d'ostéotomie (40, 41, 42, 43, 20, 21, 22, 23, 24) pourvu d'une pluralité d'éléments de guidage pour fournir un guidage d'ostéotomie, et le dispositif de contrôle comprend au moins un repère de contrôle (16) configuré pour une fixation amovible au bloc de guidage d'ostéotomie (40, 41, 42, 43, 20, 21, 22, 23, 24) ;
le dispositif de suivi (6) est configuré pour enregistrer des informations de position initiales du marqueur de repère de contrôle (16) par rapport au marqueur de repère de guidage (3) avant la livraison depuis l'usine et pour enregistrer des informations concernant la position actuelle du marqueur de repère de contrôle (16) par rapport au marqueur de repère de guidage (3) après chaque utilisation chirurgicale ; et
le dispositif de commande est configuré pour déterminer s'il existe une correspondance entre les informations de position initiale du marqueur de repère de contrôle (16) et les informations concernant la position actuelle du marqueur de repère de contrôle (16), s'il y a une correspondance, déterminer que le le guide d'ostéotomie (4) et/ou le marqueur de repère de guide (3) a/n'ont pas subi de déformation, et s'il n'y a pas de correspondance, déterminer que le guide d'ostéotomie (4) et/ou le marqueur de repère de guide (3) a/ ont subi une déformation.

2. Système de chirurgie orthopédique selon la revendication 1, comprenant en outre un dispositif de correction pour corriger la position du guide d'ostéotomie (4) par rapport au marqueur de repère de guide (3) lorsque le dispositif de contrôle détermine que le guide d'ostéotomie (4) et/ ou le repère de repère de guidage (3) a/n'a pas subi de déformation, le dispositif de correction comprenant au moins deux repères de repère de correction configurés chacun pour une fixation amovible au bloc de guidage d'ostéotomie (40, 41, 42, 43, 20, 21, 22, 23, 24),
dans lequel le dispositif de suivi (6) est configuré pour enregistrer des informations de position des au moins deux marqueurs de repère de correction par rapport au marqueur de repère de guidage (3), et
dans lequel le dispositif de commande est configuré pour dériver les informations concernant la position actuelle du guide d'ostéotomie (4) par rapport au marqueur de repère de guide (3) à partir des informations de position des au moins deux marqueurs de repère de correction par rapport au marqueur de repère de guide (3) et mettre à jour la position du guide d'ostéotomie (4) par rapport au marqueur de repère de guide (3) à l'aide des informations dérivées concernant la position actuelle.

3. Système de chirurgie orthopédique selon la revendication 2, dans lequel les informations de position des deux marqueurs de repère correcteurs par rapport au marqueur de repère de guidage (3) sont respectivement notées *T*₁ et *T*₂, *T*₁ sont représentées par une représentation de position de l'un des deux marqueurs de repère correcteurs dans un système de coordonnées du repère de repère de guidage (3) et *T*₂ est représenté par une représentation de position de l'autre des deux repères de repère de correction dans le système de coordonnées du repère de repère de guidage (3),
le dispositif de commande est configuré pour dériver une représentation de position *T*₀ d'un centre d'un bloc de guidage d'ostéotomie (40, 41, 42, 43, 20, 21, 22, 23, 24) dans le système de coordonnées du marqueur de repère de guidage (3) et une représentation positionnelle *T*₃ d'une surface du bloc guide d'ostéotomie (40, 41, 42, 43, 20, 21, 22, 23, 24) dans le système de coordonnées du marqueur de repère de guidage (3) à partir des représentations positionnelles *T*₁ et *T*₂ du au moins deux marqueurs de repère de correction dans le système de coordonnées du marqueur de repère de guidage (3) et déduire la position et l'orientation du guide d'ostéotomie (4) par rapport au marqueur de repère de guidage (3) à partir de la représentation de position *T*₀ du centre de l'ostéotomie bloc de guidage (40, 41, 42, 43, 20, 21, 22, 23, 24) dans le système de coordonnées du marqueur de repère de guidage (3) et la représentation de position *T*₃ de la surface du bloc de guidage d'ostéotomie (40, 41, 42, 43, 20, 21, 22, 23, 24) dans le système de coordonnées du repère de repère de guidage (3), et/ou
dans lequel les informations de position du marqueur de repère de contrôle (16) par rapport au marqueur de repère de guidage (3) comprennent la position et l'orientation d'un point d'extrémité avant du marqueur de repère de contrôle (16) dans un système de coordonnées du marqueur de repère de guidage (3), et/ou
dans lequel la position de chaque marqueur de repère de correction dans le système de coordonnées du marqueur de repère de guidage (3) comprend :
la position et l'orientation d'un point d'extrémité avant du marqueur de repère de correction dans le système de coordonnées du marqueur de repère de guidage (3) ; et
la position et l'orientation d'une surface de marche du marqueur de repère de correction dans le système de coordonnées du marqueur de repère de guidage (3), et
dans lequel la surface de marche est une marche du marqueur de repère de correction et est parallèle à une surface du bloc de guidage d'ostéotomie (40, 41, 42, 43, 20, 21, 22, 23, 24).

4. Système de chirurgie orthopédique selon la revendication 1, dans lequel le dispositif de navigation comprend en outre un marqueur de repère de base (15) qui est maintenu stationnaire, et la position du marqueur de repère de guidage (3) est par rapport à celle du marqueur de repère de base (15), et/ou
le dispositif de commande étant configuré pour fournir un trajet de déplacement souhaité défini par une pluralité de points de position et pour commander le bras robotique (2) pour qu'il se déplace de telle sorte que le marqueur de repère de guidage (3) suive le trajet de déplacement souhaité pour atteindre la position souhaitée.

5. Système de chirurgie orthopédique selon la revendication 1, comprenant en outre un dispositif de stockage pour stocker une cartographie de position et d'orientation entre le marqueur de repère de guidage (3) et le guide d'ostéotomie (4).

6. Système de chirurgie orthopédique selon la revendication 5, dans lequel le guide d'ostéotomie (4) comprend un bloc de guidage d'ostéotomie (40, 41, 42, 43, 20, 21, 22, 23, 24) pourvu d'un certain nombre d'éléments de guidage pour fournir de multiples modes de guidage d'ostéotomie, et dans lequel le mappage de position et d'orientation entre le marqueur de repère de guide (3) et le guide d'ostéotomie (4) comprend des mappages de position et d'orientation entre les éléments de guidage et le marqueur de repère de guide (3).

7. Système de chirurgie orthopédique selon la revendication 6, dans lequel la cartographie de position et d'orientation entre chacune des caractéristiques de guidage et le marqueur de repère de guidage (3) est déterminée par :
déterminer une relation de position relative entre les billes du marqueur de repère (3) sur le marqueur de repère de guidage (3) et établir un système de coordonnées du marqueur de repère de guidage (3) sur la base de la relation de position relative ;
déterminer des informations de position d'un centre d'un bloc de guidage d'ostéotomie (40, 41, 42, 43, 20, 21, 22, 23, 24) dans le système de coordonnées du marqueur de repère de guidage (3) ; et
déterminer la position et l'orientation de l'élément de guidage dans le système de coordonnées du marqueur de repère de guidage (3) à partir des deux informations de position de l'élément de guidage par rapport au centre du bloc de guidage d'ostéotomie (40, 41, 42, 43, 20, 21, 22, 23, 24) et les informations de position du centre du bloc de guidage d'ostéotomie (40, 41, 42, 43, 20, 21, 22, 23, 24) dans le système de coordonnées du marqueur de repère de guidage (3).

8. Système de chirurgie orthopédique selon la revendication 1, dans lequel le bloc de guidage d'ostéotomie (40, 41, 42, 43, 20, 21, 22, 23, 24) est pourvu d'au moins un trou de contrôle (409) auquel le le ou les marqueurs de repère de contrôle respectifs sont fixés, et dans lequel chaque marqueur de repère de contrôle (16) présente une marche avec une surface de marche qui est parallèle à une surface du bloc de guidage d'ostéotomie (40, 41, 42, 43, 20, 21, 22, 23, 24).

9. Système de chirurgie orthopédique selon la revendication 8, dans lequel le bloc de guidage d'ostéotomie (40, 41, 42, 43, 20, 21, 22, 23, 24) présente une surface supérieure dans laquelle sont prévus les éléments de guidage, dans lequel le contrôle un trou (409) est prévu verticalement dans la surface supérieure, et dans lequel la surface de marche correspond à la surface supérieure, et/ou
dans lequel le bloc de guidage d'ostéotomie (40, 41, 42, 43, 20, 21, 22, 23, 24) est pourvu d'un trou de correction (414a, 414b), et dans lequel deux marqueurs de repère de correction sont respectivement fixés au trou de correction (414a, 414b), ou deux repères de correction sont respectivement fixés à un trou de contrôle (409) et au trou de correction (414a, 414b).

10. Système de chirurgie orthopédique selon la revendication 9, dans lequel une ligne d'axe du trou de contrôle (409) est située dans un plan de symétrie du bloc guide d'ostéotomie (40, 41, 42, 43, 20, 21, 22, 23, 24), et dans lequel une face d'extrémité du trou de contrôle (409), la surface supérieure du bloc de guidage d'ostéotomie (40, 41, 42, 43, 20, 21, 22, 23, 24) et la surface de marche sont coplanaire.

11. Système de chirurgie orthopédique selon la revendication 8, dans lequel le bloc de guidage d'ostéotomie (40, 41, 42, 43, 20, 21, 22, 23, 24) est pourvu d'un trou de correction (414a, 414b), et dans lequel deux repères de correction sont respectivement fixés au trou de correction (414a, 414b), ou deux repères de correction sont respectivement fixés à un trou de contrôle (409) et au trou de correction (414a, 414b),
dans lequel chaque trou de correction (414a, 414b) possède une face d'extrémité qui est coplanaire à la fois avec la surface du bloc de guidage d'ostéotomie (40, 41, 42, 43, 20, 21, 22, 23, 24) et une surface en gradin de chaque marqueur de repère correcteur.

12. Système de chirurgie orthopédique selon la revendication 1, dans lequel le guide d'ostéotomie (4) comprend un bloc de guidage d'ostéotomie (40, 41, 42, 43, 20, 21, 22, 23, 24) et une interface de montage (401), l'interface de montage (401) reliée au bloc de guidage d'ostéotomie (40, 41, 42, 43, 20, 21, 22, 23, 24) par un arbre de liaison, le bloc de guidage d'ostéotomie (40, 41, 42, 43, 20, 21, 22, 23, 24) pourvu d'une pluralité d'éléments de guidage pour fournir un guidage d'ostéotomie, l'interface de montage (401) étant fixée de manière amovible à l'extrémité du bras robotique (2).

13. Système de chirurgie orthopédique selon la revendication 12, dans lequel l'arbre de liaison est une manivelle excentrique (403) qui est reliée à la fois au bloc guide d'ostéotomie (40, 41, 42, 43, 20, 21, 22, 23, 24) et l'interface de montage (401) de sorte qu'un axe de rotation de l'interface de montage (401) soit décalé par rapport à un axe de rotation du bloc de guidage d'ostéotomie (40, 41, 42, 43, 20, 21, 22, 23, 24).

14. Système de chirurgie orthopédique selon la revendication 13, dans lequel le guide d'ostéotomie (4) comprend en outre un support (402), dans lequel le bloc de guidage d'ostéotomie (40, 41, 42, 43, 20, 21, 22, 23, 24), la manivelle excentrique (403), le support (402) et l'interface de montage (401) sont connectés ensemble en séquence, et dans lequel le support (402) est pourvu de trous de montage de marqueur de repère (4021), et/ou
dans lequel le bloc de guidage d'ostéotomie (40, 41, 42, 43, 20, 21, 22, 23, 24) est fixé de manière amovible à la manivelle excentrique (403).

15. Système de chirurgie orthopédique selon la revendication 12, dans lequel les éléments de guidage comprennent un certain nombre de fentes de guidage prévues sur une seule surface du bloc de guidage d'ostéotomie (40, 41, 42, 43, 20, 21, 22, 23, 24), ou sur une surface différente autour d'une ligne d'axe du bloc de guidage d'ostéotomie (40, 41, 42, 43, 20, 21, 22, 23, 24).

16. Système de chirurgie orthopédique selon la revendication 13, dans lequel le guide d'ostéotomie (4) comprend deux blocs de guidage d'ostéotomie (40, 41, 42, 43, 20, 21, 22, 23, 24) en miroir l'un par rapport à l'autre et disposés chacun dessus avec une interface de montage (401) configurée pour une connexion amovible avec la manivelle excentrique (403), ou
dans lequel le guide d'ostéotomie (4) comprend un bloc de guidage d'ostéotomie (40, 41, 42, 43, 20, 21, 22, 23, 24) pourvu de deux interfaces de montage (401) disposées en opposition l'une par rapport à l'autre, et dans lequel le la manivelle excentrique (403) est reliée sélectivement de manière amovible à l'une des interfaces de montage (401).

17. Système de chirurgie orthopédique selon la revendication 1, comprenant en outre un sac stérile (31) disposé sur l'extrémité du bras robotique (2) de sorte qu'une partie d'extrémité du sac stérile (31) recouvre un port de connexion du bras robotique (2), le port de connexion étant connecté de manière amovible au guide d'ostéotomie (4).

18. Système de chirurgie orthopédique selon la revendication 17, dans lequel le sac stérile (31) est pourvu à une extrémité de celui-ci de trous de passage (311).

19. Guide d'ostéotomie (4) destiné à être utilisé dans le système de chirurgie orthopédique selon la revendication 1, le guide d'ostéotomie (4) comprenant un bloc de guidage d'ostéotomie (40, 41, 42, 43, 20, 21, 22, 23, 24), un arbre de liaison et une interface de montage (401), le bloc de guidage d'ostéotomie (40, 41, 42, 43, 20, 21, 22, 23, 24) étant pourvu d'un certain nombre d'éléments de guidage pour fournir de multiples modes de guidage d'ostéotomie, les deux extrémités de la tige de liaison reliées à la fois au bloc de guidage d'ostéotomie (40, 41, 42, 43, 20, 21, 22, 23, 24) et à l'interface de montage (401), l'interface de montage (401) étant configurée pour une connexion amovible avec une extrémité d'un bras robotique (2).

20. Guide d'ostéotomie (4) selon la revendication 19, dans lequel les caractéristiques de guidage comprennent une ou plusieurs fentes de guidage et un ou plusieurs trous de guidage (231, 241, 251, 271), et/ou
dans lequel l'arbre de liaison est une manivelle excentrique (403).

21. Guide d'ostéotomie (4) selon la revendication 20, dans lequel chaque fente de guidage s'étend à travers une surface du bloc de guidage d'ostéotomie (40, 41, 42, 43, 20, 21, 22, 23, 24) et présente ainsi des ouvertures d'extrémité (281), et/ou dans lequel chaque fente de guidage est de forme évasée, et/ou
dans lequel une pluralité de fentes de guidage sont incluses et disposées sur différentes surfaces autour d'une ligne d'axe du bloc de guidage d'ostéotomie (40, 41, 42, 43, 20, 21, 22, 23, 24).

22. Guide d'ostéotomie (4) selon la revendication 20, dans lequel la ou les fentes de guidage comprennent au moins une parmi une ou plusieurs fentes de guidage à 0° (407, 411), une ou plusieurs fentes de guidage à 45° (408, 410) et une ou plusieurs fentes de guidage de coupe trochléaire (221), et dans lequel le ou les trous de guidage comprennent au moins l'un des trous de guidage pour le montage d'une prothèse fémorale (404), trou de guidage(s) pour la localisation de l'outillage tibial gauche (406), trou(s) de guidage pour la localisation de l'outillage tibial droit (413) et trou(s) de fixation pour le guide d'ostéotomie (4), ou
la ou les fentes de guidage comprenant deux fentes de guidage à 0° (201, 211), deux fentes de guidage à 45° et deux fentes de guidage de coupe trochléaire (221).

23. Guide d'ostéotomie (4) selon la revendication 19, dans lequel le bloc guide d'ostéotomie (40, 41, 42, 43, 20, 21, 22, 23, 24) est une structure axisymétrique, et/ou
dans lequel le bloc de guidage d'ostéotomie (40, 41, 42, 43, 20, 21, 22, 23, 24) est pourvu d'un trou de contrôle (409) et/ou d'un trou de correction (414a, 414b).
